# FASCICULE DE BREVET EUROPEEN

(11) **EP 2 350 018 B1**
(45) Date de publication et mention de la délivrance du brevet: **07.06.2017**
(21) Numéro de dépôt: 09768167.0
(22) Date de dépôt: 10.11.2009
(51) Int. Cl.: C07D 233/54, A61K 31/4164

(54) **INHIBITEURS DE L'HISTIDINOL DESHYDROGENASE, ET LEUR UTILISATION EN TANT QUE MEDICAMENTS**
HISTIDINOLDEHYDROGENASEINHIBITOREN UND DEREN VERWENDUNG ALS MEDIKAMENTE
HISTIDINOL DEHYDROGENASE INHIBITORS, AND USE THEREOF AS MEDICAMENTS

(30) Priorité: 12.11.2008 FR 0806286
(43) Date de publication de la demande: 03.08.2011
(73) Titulaire: CENTRE NATIONAL DE LA RECHERCHE SCIENTIFIQUE, 75794 Paris (FR)
(72) Inventeur: WINUM, Jean-Yves, F-34725 Saint Andre de Sangonis (FR); MONTERO, Jean-Louis, F-34270 Lauret (FR); KÖHLER, Stephan, F-34980 Saint Clement de Riviere (FR)
(74) Mandataire: Grosset-Fournier, Chantal Catherine
(86) Numéro de dépôt international: PCT/FR2009/052159
(87) Numéro de publication internationale: WO 2010/055254

(56) Documents cités:
- JOSEPH, PASCALE ET AL: "Targeting of the Brucella suis virulence factor histidinol dehydrogenase by histidinol analogues results in inhibition of intramacrophagic multiplication of the pathogen" ANTIMICROBIAL AGENTS AND CHEMOTHERAPY , 51(10), 3752-3755 CODEN: AMACCQ; ISSN: 0066-4804, 2007, XP002518058 cité dans la demande
- ABDO, MARIE-ROSE ET AL: "Brucella suis histidinol dehydrogenase: Synthesis and inhibition studies of a series of substituted benzylic ketones derived from histidine" BIOORGANIC & MEDICINAL CHEMISTRY , 15(13), 4427-4433 CODEN: BMECEP; ISSN: 0968-0896, 2007, XP022093284 cité dans la demande
- DANCER, JANE E. ET AL: "Synthesis of potent inhibitors of histidinol dehydrogenase" BIOORGANIC & MEDICINAL CHEMISTRY LETTERS , 6(17), 2131-2136 CODEN: BMCLE8; ISSN: 0960-894X, 1996, XP004135671

## Description

La présente invention a pour objet des inhibiteurs de l'histidinol déshydrogénase. La présente invention concerne également l'utilisation de ces inhibiteurs de l'histidinol déshydrogénase en tant que médicaments.

La brucellose, également appelée fièvre de Malte, fièvre sudoro-algique, fièvre ondulante, mélitococcie ou fièvre méditerranéenne est une anthropozoonose (maladie transmise à l'homme par les animaux) due à des coccobacilles du genre *Brucella.*

La brucellose humaine, bien que devenue rare en France depuis la mise en place de mesures prophylactiques sévères en 1978, reste une maladie pouvant entraîner des complications graves si un traitement n'est pas rapidement mis en place. Comme pour toute maladie infectieuse, la prévention (surveillance et éradication de la maladie chez le bétail) reste le meilleur moyen de lutte.

Les bactéries du genre *Brucella* sont de petits coccobacilles à Gram négatif, non encapsulés, non sporulés et aérobie stricte.

Le mécanisme du pouvoir pathogène de *Brucella* reste encore mal connu. La bactérie est phagocytée par les macrophages et se développe dans le phagosome en inhibant la fusion de ce dernier avec les lysosomes. La bactérie peut ainsi échapper au système immunitaire et entretenir la chronicité de la maladie.

De plus, la bactérie synthétise des protéines dites « de choc septique » responsables de la phase aiguë de la maladie.

Chez l'homme, certaines espèces de *Brucella* provoquent une infection généralisée avec état septique ; des localisations viscérales ou ostéo-articulaires subséquentes sont possibles. La maladie passe généralement par une phase aiguë durant laquelle les bactéries sont décelables dans le sang. Elle a également une forte tendance à passer à la chronicité, les bactéries se logeant dans le système réticulo-endothélial (foie, rate, moelle osseuse, ganglions) où leur position intracellulaire dans les macrophages les met à l'abri du système immunitaire et des traitements [Franco et al., Lancet Infect Dis 7 (2007): 775-786]*.*

Il n'existe à ce jour aucun vaccin efficace contre la brucellose chez l'homme.

La brucellose se traite donc avec des antibiotiques. Il est important de mettre en place un traitement rapide pour éviter une infection chronique. Comme *Brucella* est une bactérie intracellulaire, il est nécessaire d'utiliser des antibiotiques à la fois actifs sur la bactérie et capables de passer la barrière de la membrane plasmique des macrophages. Les tétracyclines et la rifampicine sont souvent associées à la streptomycine, au chloramphénicol et aux sulfamidés pour traiter la maladie.

Comme dans beaucoup d'infections bactériennes traitées par des multi antibiothérapies, des exemples de résistances de *Brucella* aux antibiotiques ont été démontrés chez l'homme [Baikam et al., Int. J. Antimicrob. Agents 23 (2004): 405-407*;* Kinsara et al., Antimicrob. Agents Chemother. 43 (1999): 1531*;* Memish et al., J. Infect. 40 (2000): 59-63]*.* Cette capacité de résistance spontanée, ainsi que le degré d'infectiosité très élevé par aérosols, a conduit les spécialistes à considérer *Brucella* comme un agent potentiel de bioterrorisme et des souches résistantes aux antibiotiques peuvent être facilement construites.

Ainsi, il est important de développer de nouvelles molécules permettant d'éradiquer les bactéries, lorsqu'elles sont résistantes aux antibiotiques classiquement utilisés.

Plusieurs équipes ont étudié le « virulome » de *Brucella* afin d'identifier les facteurs impliqués dans la pathogénicité de la bactérie [Köhler et al., Proc. Natl. Acad. Sci USA 99 (2002): 15711-15716*;* Delrue et al., FEMS Microbiol. Lett. 231 (2004): 1-12*].*

Parmi les facteurs découverts, l'enzyme histidinol déshydrogénase (HDH, EC. 1.1.1.23) a été montrée comme essentielle pour la croissance de la bactérie à l'état intracellulaire, et donc de son pouvoir infectieux. L'HDH, dernière enzyme de la chaine de biosynthèse de l'histidine, est retrouvée chez les bactéries, les plantes et les eucaryotes inférieurs tels que les levures, mais n'est pas retrouvée exprimée à ce jour chez les mammifères [Nagai et al., Proc. Natl. Acad. 88 (1991): 4133-4137*].*

Du fait de son absence d'équivalent chez les mammifères, l'HDH constitue une cible thérapeutique pour le développement de traitements anti-infectieux contre les pathogènes à développement intracellulaire dépendant de l'histidine.

Plusieurs molécules visant à inhiber l'activité de cette enzyme ont été développées et sont décrites dans l'art antérieur.

Abdo *et al.* [Abdo et al. Bioorg Med Chem 15 (2007): 4427-4433] ont décrit des dérivés de benzyle cétone de l'histidine, capables d'inhiber *in vitro* l'histidinol déshydrogénase.

Abdo *et al.* [Abdo et al. J Enzyme Inhib Med Chem 23 (2008): 357-361] ont également décrit des dérivés phényle sulphonyle hydrazine de l'histidine, capables *d'inhiber in vitro* l'histidinol déshydrogénase.

Joseph *et al.* [Joseph et al. Antimicrob Agents Chemother 51 (2007): 3752-3755] ont montré que les dérivés benzyle cétone de l'histidine inhibant l'histidinol déshydrogénase in *vitro,* inhibent la prolifération des bactéries en milieu liquide. Les auteurs de ce document ont également montré que les dérivés benzyle cétone de l'histidine sont capables de réduire la prolifération des bactéries lorsqu'elles sont entrées dans le macrophage. Cependant, parmi tous les inhibiteurs décrits dans ce document, seuls les dérivés benzyle cétone de l'histidine ayant une affinité élevée pour l'enzyme exercent un effet inhibiteur fort sur la prolifération des bactéries dans le macrophage.

Dancer *et al.* [Dancer et al. Bioorg Med Chem 6 (1996): 2131-2136*]* ont également décrit des dérivés benzyle cétone de l'histidine, capables d'inhiber *in vitro* l'histidinol déshydrogénase.

Ainsi, il existe un réel besoin de fournir de nouvelles molécules capables d'inhiber à faible dose l'histidinol déshydrogénase, et inhibant efficacement la prolifération des bactéries dans le macrophage.

Les Inventeurs ont découvert de manière inattendue que les molécules de formule générale (I) mentionnée ci après inhibent *in vitro* et *in vivo* l'histidinol déshydrogénase.

L'invention a pour but de fournir de nouvelles molécules inhibant l'histidinol déshydrogénase.

Un autre but de l'invention est de fournir des médicaments capables d'éradiquer les bactéries ayant infecté le macrophage.

Dans le cadre de l'invention, « un groupement hétérocyclique en C₅-C₁₀» correspond à tout cycle carboné comprenant un squelette de 5, ou 6 ou 7, ou 8, ou 9 ou 10 atomes de carbones. Dans ledit cycle, un ou plusieurs atomes de carbones pouvant être substitués par d'autres atomes différents du carbone, et préférentiellement par un atome de la famille des chalcogènes ou des pnictogènes.

La famille des chalcogènes comprend l'oxygène (O), le soufre (S), le sélénium (Se), le tellure (Te), le polonium (Po) radioactif et un élément de synthèse le ununhexium (Uuh). Préférentiellement, l'oxygène, le soufre et le sélénium sont choisis comme atomes substituants.

La famille des pnictogènes comprend l'azote (N), le phosphore (P), l'arsenic (As), l'antimoine (Sb), le bismuth (Bi) et l'ununpentium (Uup). Préférentiellement, l'azote et le phosphore sont choisis comme atomes substituants.

Lesdits hétérocycles de l'invention sont préférentiellement en C₅-C₆, c'est-à-dire qu'ils possèdent un squelette de base de 5 ou 6 atomes.

Ces hétérocycles peuvent être saturés ou insaturés, et ainsi comporter de aucune à 3 doubles liaisons.

Les hétérocycles préférés de l'invention sont des hétérocycles insaturés en C₅-C₆ choisis parmi la pyrrolidine, le tétrahydrothiophène, le tétrahydrofurane, la pipéridine, le pyrane, le dioxane, la morpholine, le pyrole, le thiophène, le furane, la pyridine, la pyrimidine la pyrazine, la triazine, l'imidazole, le thiazole et l'oxazole, la purine, le triazole, le tétrazole, le thiadiazole, et le benzothiazole.

Dans le cadre de l'invention, Y, lorsqu'il représente un groupe alkylène linéaire ou ramifié saturé, peut être choisi dans le groupe comprenant : le méthylène (-CH₂-), l'éthylène (-CH₂-CH₂-), le propylène (-CH₂-CH₂-CH₂-), le 1-méthyléthylène (-CH₂(CH₃)-CH₂-), le 2-méthyléthyléne (-CH₂-CH₂(CH₃)-), le 1,1-diméthylméthylène (-C(CH₃)₂-), le butyléne (-CH₂-CH₂-CH₂-CH₂-), le 1-méthylpropylène (-CH (CH₃)-CH₂-CH₂-), le 2-méthylpropylène (-CH₂-CH(CH₃)-CH₂-), le 3-méthylpropylène (-CH₂-CH₂-CH(CH₃)-), le 1,2-diméthylétylène (-CH(CH₃)-CH(CH₃)-), le 1,1-diméthyléthylène (-C(CH₃)₂-CH₂-), le 2,2-diméthyléthylène (-CH₂-C(CH₃)₂-), le 1-éthyléthylène (-CH(C₂H₅)-CH₂-), le 2-éthyléthylène (-CH₂-CH(C₂H₅)-), le 1-éthyle 1-méthylméthylène (-C(C₂H₅)(CH₃)-), le pentylène (-CH₂-CH₂-CH₂-CH₂- CH₂-), le 1-méthylbutylène (-CH (C₃)-CH₂-CH₂-CH₂), le 2- méthylbutylène (-CH₂-CH(CH₃)-CH₂-CH₂), le 3- méthylbutylène (-CH₂-CH₂₋CH(CH₃)-CH₂), le 4- méthylbutylène (-CH₂-CH₂-CH₂-CH(CH₃)-), le 1,1-diméthylpropylène (-C(CH₃)₂-CH₂-CH₂-), le 2,2-diméthylpropylène (-CH₂-C(CH₃)₂-CH₂-), le 3,3-diméthylpropylène (-CH₂-CH₂-C(CH₃)₂-), le 1,2-diméthylpropylène (-CH(CH₃)-CH(CH₃)-CH₂-), le 1,3-diméthylpropylène (-CH(CH₃)-CH₂-CH(CH₃)-), le 2,3-diméthylpropylène (-CH₂-CH(CH₃)-CH(CH₃)-), le 1,1,2-triméthyléthylène (-C(CH₃)₂-CH(CH₃)-), le 1,2,2-triméthyléthylène (-CH(CH₃)-C(CH₃)₂-), l'hexylène (-CH₂-CH₂-CH₂-CH₂-CH₂-CH₂-), le 1-méthylpentylène (-CH(CH₃)-CH₂-CH₂-CH₂-CH₂-), le 2-méthylpentylène (-CH₂-CH(CH₃)-CH₂-CH₂-CH₂-), le 3-méthylpentylène (-CH₂-CH₂-CH (CH₃)-CH₂-CH₂-), le 4-méthylpentylène (-CH₂-CH₂-CH₂-CH(CH₃)-CH₂-), le 5-méthylpentylène (-CH₂-CH₂-CH₂-CH₂-CH(CH₃)-), le 1,2-diméthylbutylène (-CH(CH₃)-CH(CH₃)-CH₂-CH₂-), le 1,3-diméthylbutylène (-CH(CH₃)-CH₂-CH(CH₃)-CH₂-), le 1,4-diméthylbutylène (-CH(CH₃)-CH₂-CH₂-CH(CH₃)-), le 2,3-diméthylbutylène (-CH₂-CH(CH₃)-CH(CH₃)-CH₂-), le 2,4-diméthylbutylène (-CH₂-CH(CH₃)-CH₂-CH(CH₃)-), le 3,4-diméthylbutylène (-CH₂-CH₂-CH(CH₃)-CH(CH₃)-).

Lorsque Y représente un groupe alkylène linéaire ou ramifié insaturé, il peut être choisi dans le groupe comprenant : le vinylène (-CH=CH-), l'éthynylène (-C≡C-), le prop l-ènyléne (-C=CH-CH₂-), le prop 2-ènylène (-CH₂-CH=CH-), le propanediénylène (-CH=C=CH-), le prop 1-ynylène (-C≡C-CH₂-), le prop 2-ynylène (-CH₂-C≡C-), le but 1-ènylène (-CH=CH-CH₂-CH₂-), le cis ou trans but 2-ènylène (-CH₂-CH=CH-CH₂-), le but 3-ènylène (-CH₂-CH₂-CH=CH-), le but 1,2-diènylène (-CH=CH=CH-CH₂-), le but 1,3 diènylène (-CH=CH₂-CH=CH-), le but 1,2,3 triènylène (-CH=CH=CH=CH-), le but 1-ynylène (-C≡C-CH₂-CH₂-), le but 2-ynylène (-CH₂-C≡C-CH₂-), le but 3-ynylène (-CH₂-CH₂-C≡C-), le but 1,3 diynylène (-C≡C-C≡C-).

Dans le cadre de l'invention, un groupe aryle est un groupe dérivant d'un noyau aromatique. Préférentiellement, le groupe aryle correspond à un phényle ou un naphtyle.

Les cycloalkyles de l'invention en C₃-C₆ peuvent être choisis parmi les cycles suivants : le cyclopropyle, le cyclobutyle, le cyclopentyle et le cyclohéxyle.

Les atomes d'halogènes définis dans l'invention sont préférentiellement choisis parmi le fluor (F), le chlore (Cl) le brome (Br) et l'iode (I).

L'invention a pour objet des composés de formule générale suivante (Ia): 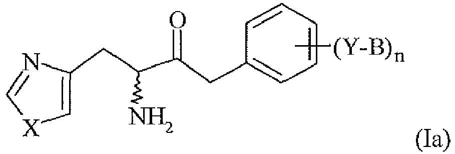 où X représente un N
caractérisés en ce que
- le groupe phényle est substitué par n groupement(s) Y-B, n variant de 1 à 5, ledit groupement Y-B étant tel que :
   - Y représente un groupe alkylène linéaire ou ramifié, saturé ou insaturé, comprenant de 1 à 6 atomes de carbone, de préférence de 2 à 4 atomes de carbone, et
   - B représente un groupe choisi parmi :
      - un groupe aryle ou hétéroaryle, notamment un groupe phényle e ou naphtyle
      - un groupement hétérocyclique en C₅-C₁₀, préférentiellement en C₅-C₆, comportant de 1 à 6 hétéroatomes choisis parmi les éléments de la famille des chalcogènes et des pnictogènes, et
      - un groupe cycloalkyle en C₃-C₆,
   ledit groupe B étant substitué par un ou plusieurs substituant(s), préférentiellement par un substituant, le(s)dit(s) substituant(s) étant choisi(s) dans le groupe comprenant :
   ∘ un atome d'halogène,
   ∘ un groupe alcoxy comprenant de 1 à 4 atomes de carbone,
   ∘ un groupe aryloxy, notamment un groupe phényle oxy
   ∘ un groupe alkyle linéaire, ramifié ou cyclique, saturé ou insaturé, comprenant de 1 à 6 atomes de carbone, de préférence de 2 à 4 atomes de carbone, éventuellement substitué par un ou plusieurs atomes d'halogènes, et
   ∘ un groupe aryle, préférentiellement un groupe phényle e, éventuellement substitué par un alkyle en C₁-C₄,
   ∘ un groupe hydroxyle,
   ∘ un groupe thiol,
   ∘ un groupe amine,
   ∘ un groupe amide,
   ∘ un groupe acide carboxylique,
   ∘ un groupe cyano,
   ∘ un groupe azoture, et
   ∘ un groupe nitro,
   lesdits composés étant sous la forme, d'un racémate, de l'un quelconque de leurs énantiomères, ou de l'un quelconque des différents tautomères correspondant aux dits racémates et énantiomères, ainsi que sous la forme de leurs sels pharmaceutiquement acceptables.

Un des modes de réalisation préféré de l'invention concerne les composés mentionnés précédemment, de formule générale (Ib) suivante :

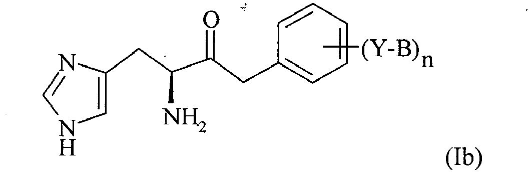

Les composés de formule générale (Ib) correspondent à un énantiomère particulier des composés de formule générale Ia, c'est-à-dire les composés de configuration S.

Ces composés sont des dérivés substitués de la L-histidine phényle.

Un autre mode de réalisation préférée de la description concerne des composés, définis par les formules générales Ia ou Ib, caractérisés en ce que, lesdits composés sont monosubstitués par un groupe Y-B, ledit groupe Y-B étant tel que :
- Y représente une liaison simple, un alkylène en C₁-C₃, un alcénylène en C₂-C₃ ou un alcynylène en C₂-C₃, et
- B représente un groupe aryle ou hétéroaryle, préférentiellement un groupe phényle, éventuellement substitué par :
   ∘ un atome d'hydrogène,
   ∘ un atome d'halogène,
   ∘ un groupe alcoxy comprenant de 1 à 4 atomes de carbone,
   ∘ un groupe aryloxy, notamment un groupe phényloxy
   ∘ un groupe alkyle linéaire, ramifié ou cyclique, saturé ou insaturé, comprenant de 1 à 6 atomes de carbone, de préférence de 2 à 4 atomes de carbone, éventuellement substitué par un ou plusieurs atomes d'halogènes, et
   ∘ un groupe aryle, préférentiellement un groupe phényle, éventuellement substitué par un alkyle en C₁-C₄.
   ∘ un groupe hydroxyle,
   ∘ un groupe thiol,
   ∘ un groupet amine, primaire secondaire ou ternaire
   ∘ un groupe amide,
   ∘ un groupe acide carboxylique, et
   ∘ un groupement cyano.
   ∘ un groupement azoture
   ∘ un groupement nitro

Un autre mode de réalisation préférée de l'invention concerne des composés, définis par les formules générales Ia ou Ib, caractérisés en ce que, lesdits composés sont monosubstitués par un groupe Y-B, ledit groupe Y-B étant tel que :
- Y représente un alkylène en C₁-C₃, un alcénylène en C₂-C₃ ou un alcynylène en C₂-C₃, et
- B représente un groupe aryle ou hétéroaryle, préférentiellement un groupe phényle, éventuellement substitué par :
   ∘ un atome d'hydrogène,
   ∘ un atome d'halogène,
   ∘ un groupe alcoxy comprenant de 1 à 4 atomes de carbone,
   ∘ un groupe aryloxy, notamment un groupe phényloxy
   ∘ un groupe alkyle linéaire, ramifié ou cyclique, saturé ou insaturé, comprenant de 1 à 6 atomes de carbone, de préférence de 2 à 4 atomes de carbone, éventuellement substitué par un ou plusieurs atomes d'halogènes, et
   ∘ un groupe aryle, préférentiellement un groupe phényle, éventuellement substitué par un alkyle en C₁-C₄.
   ∘ un groupe hydroxyle,
   ∘ un groupe thiol,
   ∘ un groupet amine, primaire secondaire ou ternaire
   ∘ un groupe amide,
   ∘ un groupe acide carboxylique, et
   ∘ un groupement cyano.
   ∘ un groupement azoture
   un groupement nitro.

Dans ledit mode de réalisation préféré de la description, le groupe Y est soit une liaison simple, soit un alkylène en C₁ (méthylène ; -CH₂-), en C₂ (éthylène ; -CH₂-CH₂-) ou C₃ (propylène ; -CH₂-CH₂-CH₂-), soit un alcénylène en C2 (vynylylène ; -CH=CH-) ou C3 (prop 1-enylène ; -CH=CH-CH₂-, ou prop 2-énylène ; -CH₂-CH=CH-), lesdits alcénylènes étant préférentiellement en configuration trans (opposé), soit un alcynylène en C2 (éthylylène ;-C=C-) ou C3 (prop 1-ynylène ; -C≡C-CH₂-, ou prop 2-ynylène ; -CH₂-C≡C-).

Dans ledit mode de réalisation préféré de l'invention, le groupe Y est soit un alkylène en C₁ (méthylène ; -CH₂-), en C₂ (éthylène ; -CH₂-CH₂-) ou C₃ (propylène ; -CH₂-CH₂-CH₂-), soit un alcénylène en C2 (vynylylène ; -CH=CH-) ou C3 (prop 1-enylène ; -CH=CH-CH₂-, ou prop 2-énylène ; -CH₂-CH=CH-), lesdits alcénylènes étant préférentiellement en configuration trans (opposé), soit un alcynylène en C2 (éthylylène ; -C≡C-) ou C3 (prop 1-ynylène ; -C≡C-CH₂-, ou prop 2-ynylène ; -CH₂-C≡C-).

Dans un mode de réalisation préféré, l'invention a pour objet les composés de formule (Ia) ou (Ib) dans lesquels le groupe Y-B est en position para du groupe phényle.

Un autre mode de réalisation de la description concerne les composés susmentionnés, où :
- Y représente une liaison simple, et
- B représente
   ▪ un groupe naphtyle ou
   ▪ un groupe phényl substitué, préférentiellement en position para, par
      ∘ un groupe alkyle linéaire, ramifié ou cyclique, saturé ou insaturé, comprenant de 1 à 6 atomes de carbone, de préférence de 2 à 4 atomes de carbone, ledit groupe alkyle étant éventuellement substitué par un ou plusieurs atomes d'halogènes,
      ∘ un groupe aryloxy, notamment un phénoxy,
      ∘ un groupe C1-C4 alcoxy,
      ∘ un halogène, ou
      ∘ un groupe phényle, éventuellement substitué par un alkyle en C₁-C₄, lesdits composés étant notamment les composés de formule Ib.2a, Ib.2b, Ib.2c, Ib.2d, Ib.2e, Ib.2f, Ib.2g, ou Ib.2h.

Un autre mode de réalisation de l'invention concerne les composés susmentionnés, où :
- Y représente un alkylène en C₁-C₃, et
- B représente un groupe phényle
lesdits composés étant notamment le composé de formule Ib.2i.

Un autre mode de réalisation de l'invention concerne les composés susmentionnés, où :
- Y représente un alcénylène en C₂-C₃, et
- B représente un groupe phényl, éventuellement substitué par
   ∘ un groupe alkyle linéaire, ramifié ou cyclique, saturé ou insaturé, comprenant de 1 à 6 atomes de carbone, de préférence de 2 à 4 atomes de carbone, éventuellement substitué par un ou plusieurs atomes d'halogènes, ou
   ∘ un groupe aryle, notamment un phényle
lesdits composés étant notamment le composé de formule Ib.2j, Ib.2k, Ib.2l ou Ib.2m.

Un autre mode de réalisation de l'invention concerne les composés susmentionnés, où :
- Y représente un alcynylène en C₂-C₃, et
- B représente un groupe phényl, eventuellement substitué par
   ∘ un groupe alkyle linéaire, ramifié ou cyclique, saturé ou insaturé, comprenant de 1 à 6 atomes de carbone, de préférence de 2 à 4 atomes de carbone, ou
   ∘ un groupe aryle, notamanent un phényle
lesdits composés étant notamment le composé de formule Ib.3a, Ib.3b, Ib.3c ou Ib.3e.

Un autre mode de réalisation préféré de la description concerne les composés susmentionnés, lesdits composés étant choisis parmi les composés suivants (les composés Ib.2a-Ib.2h ne font pas partie de l'invention):

| Formule no | Structure | nom |
|---|---|---|
| (Ib.2a) | 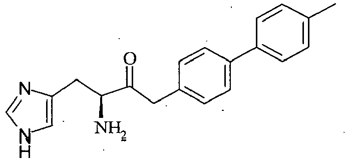 | (3*S*)-3-amino-4-(1*H-*imidazol-4-yl)-1-(4'-méthylbiphenyl-4-yl)butan-2-one |
| (Ib.2b) | 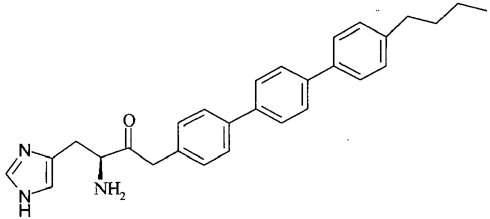 | (3*S*)-3-amino-1-(4'-butylbiphenyl-4-yl)-4-(1*H-*imidazol-4-yl) butan-2-one |
| (Ib.2c) | 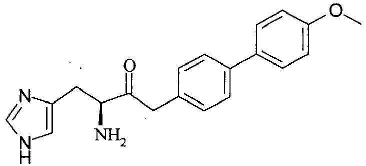 | (3*S*)-3-amino-4-(1*H-*imidazol-4-yl)-1-(4'-méthoxybiphenyl-4-yl)butan-2-one |
| (Ib.2d) | 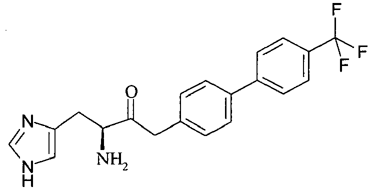 | (3*S*)-3-amino-4-(1*H-*imidazol-4-yl)-1-[4'-(trifluorométhyl) biphenyl-4-yl]butan-2-one |
| (Ib.2e) | 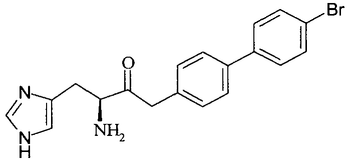 | (3*S*)-3-amino-1-(4'-bromobiphenyl-4-yl)-4-(1*H-*imidazol-4-yl)butan-2-one |
| (Ib.2f) | 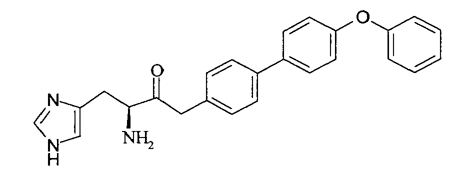 | (3*S*)-3-amino-4-(1*H-*imidazol-4-yl)-1-(4'-phenoxybiphenyl-4-yl)butan-2-one |
| (Ib.2g) | 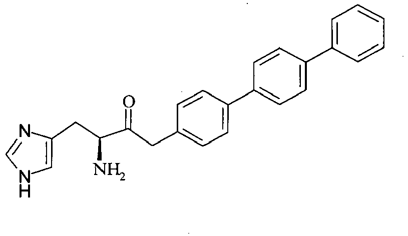 | (3*S*)-3-amino-4-(1*H-*imidazol-4-yl)-1-(1,1':4',1"-terphenyl-4-yl)butan-2-one |
| (Ib.2h) | 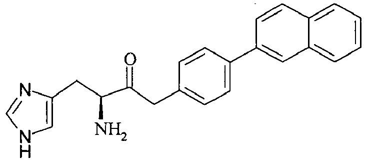 | (3*S*)-3-amino-4-(1*H-*imidazol-4-yl)-1-[4-(2-naphthyl)phenyl] butan-2-one |
| (Ib.2i) | 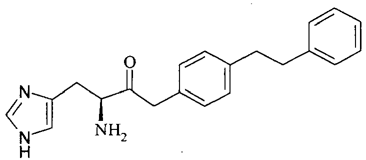 | (3*S*)-3-amino-4-(1*H-*imidazol-4-yl)-1-[4-(2-phenyléthyl)phenyl] butan-2-one |
| (Ib.2j) | 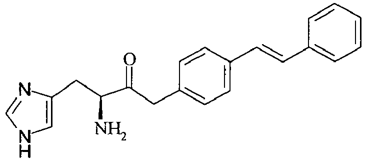 | (3*S*)-3-amino-4-(1*H-*imidazol-4-yl)-1-{4-[(*E*)-2-phenylvinyl] phenyl}butan-2-one |
| (Ib.2k) | 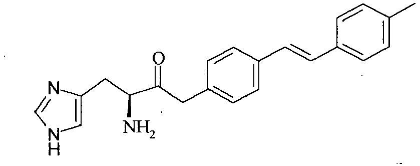 | (3*S*)-3-amino-4-(1*H-*imidazol-4-yl)-1-{4-[(*E*)-2-(4-méthylphenyl) vinyl]phenyl}butan-2-one |
| (Ib.2l) | 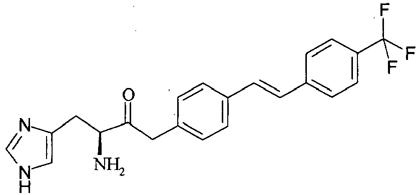 | (3*S*)-3-amino-4-(1*H-*imidazol-4-yl)-1-(4-{(*E*)-2-[4-(trifluoro méthyl)phenyl]vinyl}phe nyl) butan-2-one |
| (Ib.2m) | 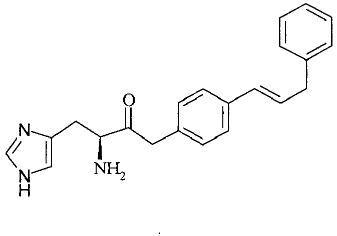 | (3*S*)-3-amino-4-(1*H-*imidazol-4-yl)-1-{4-[(1*E*)-3-phenylprop-1-èn-1-yl]phenyl}butan-2-one |
| (Ib.2n) | 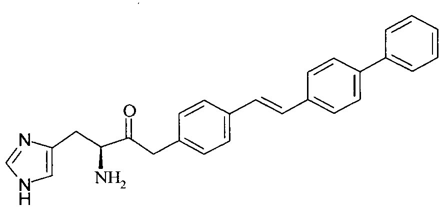 | (3*S*)-3-amino-1-{4-[(*E*)-2-biphenyl-4-ylvinyl]phenyl}-4-(1*H-*imidazol-4-yl)butan-2-one |
| (Ib.3a) | 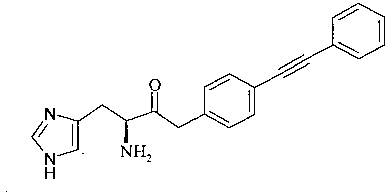 | (3*S*)-3-amino-4-(1*H-*imidazol-4-yl)-1-[4-(phenyléthynyl)phenyl] butan-2-one |
| (Ib.3b) | 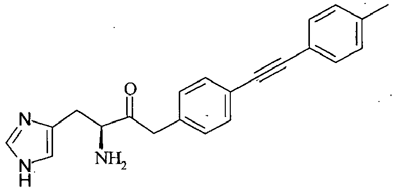 | (3*S*)-3-amino-4-(1*H-*imidazol-4-yl)-1-{4-[(4-méthylphenyl) éthynyl]phenyl} butan-2-one |
| (Ib.3c) | 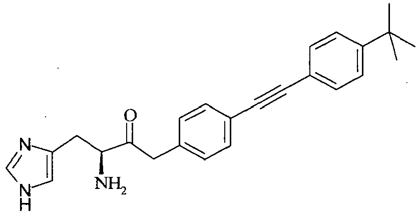 | (3*S*)-3-amino-1-{4-[(4-*tert-*butylphenyl)éthynyl]phen yl}-4-(1*H*-imidazol-4-yl)butan-2-one |
| (Ib.3d) | 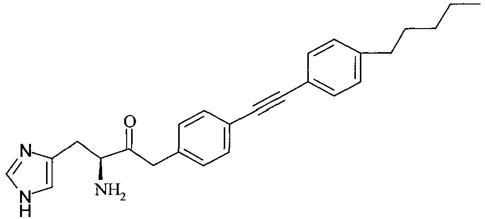 | (3*S*)-3-amino-4-(1*H-*imidazol-4-yl)-1-{4-[(4-pentylphenyl) éthynyl]phenyl} butan-2-one |
| (Ib.3e) | 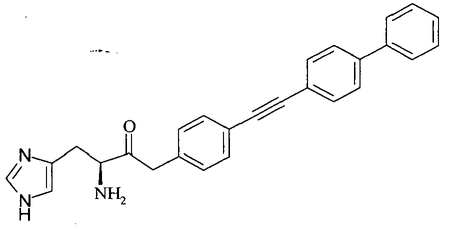 | (3*S*)-3-amino-1-[4-(biphenyl-4-yléthynyl)phenyl]-4-(1*H-*imida zol-4-yl)butan-2-one |

Un autre mode de réalisation préféré de l'invention concerne les composés susmentionnés, lesdits composés étant choisis parmi les composés suivants :

| Formule no | Structure | nom |
|---|---|---|
| (Ib.2i) | 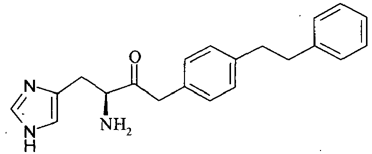 | (3*S*)-3-amino-4-(1*H-*imidazol-4-yl)-1-[4-(2-phenyléthyl)phenyl] butan-2-one |
| (Ib.2j) | 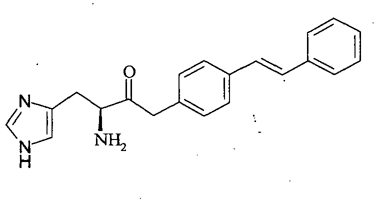 | (3*S*)-3-amino-4-(1*H-*imidazol-4-yl)-1-{4-[(*E*)-2-phenylvinyl] phenyl}butan-2-one |
| (Ib.2k) | 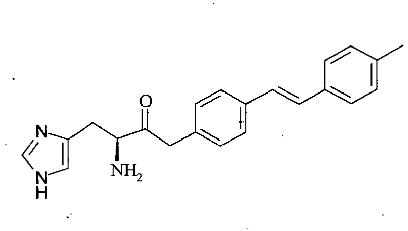 | (3*S*)-3-amino-4-(1*H-*imidazol-4-yl)-1-{4-[(*E*)-2-(4-méthylphenyl) vinyl]phenyl}butan-2-one |
| (Ib.2l) | 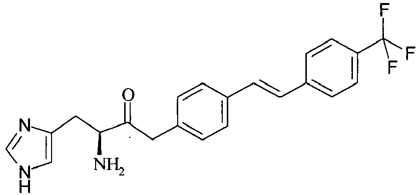 | (3*S*)-3-amino-4-(1*H-*imidazol-4-yl)-1-(4-{(*E*)-2-[4-(trifluoro méthyl)phenyl]vinyl}phe nyl) butan-2-one |
| (Ib.2m) | 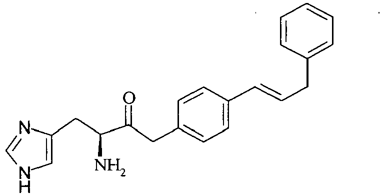 | (3*S*)-3-amino-4-(1*H-*imidazol-4-yl)-1-{4-[(1*E*)-3-phenylprop-1-èn-1-yl]phenyl}butan-2-one |
| (Ib.2n) | 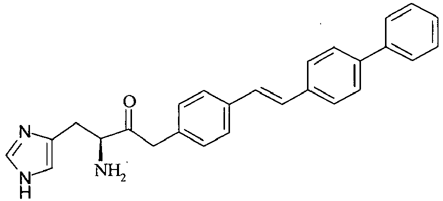 | (3*S*)-3-amino-1-{4*-*[(*E*)-2-biphenyl-4-ylvinyl]phenyl}-4-(1*H-*imidazol-4-yl)butan-2-one |
| (Ib.3a) | 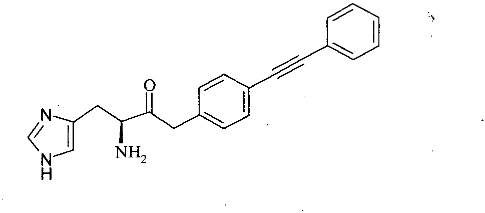 | (3*S*)-3-amino-4-(1*H-*imidazol-4-yl)-1-[4-(phenyléthynyl)phenyl] butan-2-one |
| (Ib.3b) | 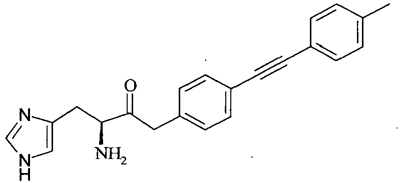 | (3*S*)-3-amino-4-(1*H-*imidazol-4-yl)-1-{4-[(4-méthylphenyl) éthynyl]phenyl}butan-2-one |
| (Ib.3c) | 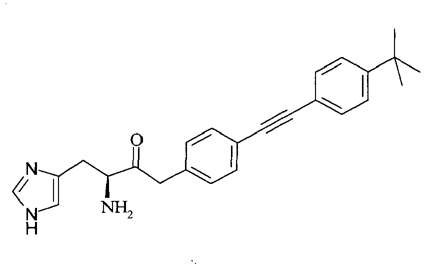 | (3*S*)-3-amino-1-{4-[(4-*tert-*butylphenyl)éthynyl]phen yl}-4-(1*H-*imidazol-4-yl)butan-2-one |
| (Ib.3d) | 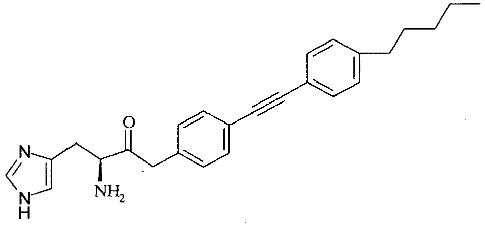 | (3*S*)-3-amino-4-(1*H-*imidazol-4-yl)-1-{4-[(4-pentylphenyl) éthynyl]phenyl}butan-2-one |
| (Ib.3e) | 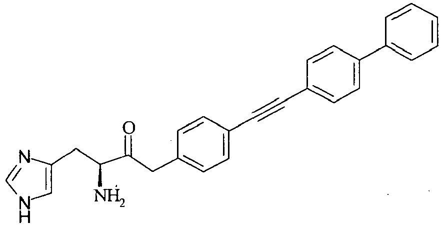 | (3*S*)-3-amino-1-[4-(biphenyl-4-yléthynyl)phenyl]-4-(1*H-*imida zol-4-yl)butan-2-one |

Les composés selon l'invention peuvent être synthétisés par tout procédé connu de l'homme de métier, à partir de produits disponibles dans le commerce. La synthèse de certains composés de l'invention est présentée dans les exemples ci-après.

L'invention a également pour objet les composés précédents, à titre de médicament.

Egalement, l'un des modes de réalisation avantageux de l'invention concerne des compositions pharmaceutiques comprenant à titre de substance active au moins un des composés précédents, en association avec un véhicule pharmaceutiquement acceptable.

Les compositions avantageuses selon l'invention sont caractérisées en ce qu'elle inhibent l'activité de l'histidinol deshydrogénase et inhibent la prolifération intracellulaire des bactéries. La mesure de l'inhibition de l'activité de l'enzyme et de la prolifération est réalisée selon les tests décrits dans la partie expérimentale. L'inhibition de l'histidinol deshydrogénase est évaluée par la détermination de l'inhibition de 50% de l'activité de conversion de l'enzyme à une concentration donnée de l'inhibiteur, et l'inhibition de la prolifération est évaluée par la mesure de la diminution (en fonction de la concentration de l'inhibiteur) de la prolifération comparée à la prolifération en absence de composés inhibiteurs.

Le dosage de la substance active dépend particulièrement du mode d'administration, et est aisément déterminé par l'homme de métier.

Par exemple, sans pour autant être limitatif, les compositions telles que définies dans l'invention sont caractérisées en ce qu'elles sont susceptibles d'être administrées par voie intraveineuse à raison de 5 à 90 µg/kg/jour chez l'homme.

Un des modes de réalisation préféré de l'invention concerne des composés ou des compositions pharmaceutiques susmentionnés, pour le traitement ou la prévention d'infections causées par des microorganismes, notamment des bactéries ou des champignons tels que les levures.

Dans un mode de réalisation avantageux de l'invention, lesdits microorganismes sont des microorganismes exprimant l'enzyme histidinol déshydrogénase.

Dans un mode de réalisation préféré de l'invention, lesdits microorganismes sont des bactéries, notamment des bactéries des genres *Brucella* ou *Mycobacterium.*

Dans le cadre de l'invention, les bactéries du genre *Brucella* sont représentées par : *Brucella abortus, Brucella canis, Brucella melitensis, Brucella neotomae, Brucella ovis, Brucella suis, Brucella pinnipediae, Brucella cetaceae et Brucella microti.*
De plus, dans le cadre de l'invention, les bactéries du genre Mycobacterium sont représentées par : *Mycobacterium abscessus, Mycobacterium africanum, Mycobacterium agri, Mycobacterium aichiense, Mycobacterium alvei, Mycobacterium arupense, Mycobacterium asiaticum, Mycobacterium aubagnense, Mycobacterium aurum, Mycobacterium austroafricanum, Mycobacterium avium complex (MAC),* comprenant, *Mycobacterium avium, Mycobacterium avium paratuberculosis,, Mycobacterium avium silvaticum, Mycobacterium avium "hominissuis", Mycobacterium colombiense, Mycobacterium boenickei, Mycobacterium bohemicum, Mycobacterium bolletii, Mycobacterium botniense, Mycobacterium bovis, Mycobacterium branderi, Mycobacterium brisbanense, Mycobacterium brumae, Mycobacterium canariasense, Mycobacterium caprae, Mycobacterium celatum, Mycobacterium chelonae,, Mycobacterium chimaera, Mycobacterium chitae, Mycobacterium chlorophenolicum, Mycobacterium chubuense, Mycobacterium conceptionense, Mycobacterium confluentis, Mycobacterium conspicuum, Mycobacterium cookii, Mycobacterium cosmeticum, Mycobacterium diernhoferi, Mycobacterium doricum, Mycobacterium duvalii, Mycobacterium elephantis, Mycobacterium fallax, Mycobacterium farcinogenes, Mycobacterium flavescens, Mycobacterium florentinum, Mycobacterium fluoroanthenivorans, Mycobacterium fortuitum, Mycobacterium fortuitum subsp. acetamidolyticum, Mycobacterium frederiksbergense, Mycobacterium gadium, Mycobacterium gastri, Mycobacterium genavense, Mycobacterium gilvum, Mycobacterium goodii, Mycobacterium gordonae, Mycobacterium haemophilum, Mycobacterium hassiacum, Mycobacterium heckeshornense, Mycobacterium heidelbergense, Mycobacterium hiberniae, Mycobacterium hodleri, Mycobacterium holsaticum, Mycobacterium houstonense, Mycobacterium immunogenum, Mycobacterium interjectum, Mycobacterium intermedium, Mycobacterium intracellulare, Mycobacterium kansasii, Mycobacterium komossense, Mycobacterium kubicae, Mycobacterium kumamotonense, Mycobacterium lacus, Mycobacterium lentiflavum, Mycobacterium leprae, Mycobacterium lepraemurium, Mycobacterium madagascariense, Mycobacterium mageritense, Mycobacterium malmoense, Mycobacterium marinum, Mycobacterium massiliense, Mycobacterium microti, Mycobacterium monacense, Mycobacterium montefiorense, Mycobacterium moriokaense, Mycobacterium mucogenicum, Mycobacterium murale, Mycobacterium nebraskense, Mycobacterium neoaurum, Mycobacterium neworleansense, Mycobacterium nonchromogenicum, Mycobacterium novocastrense, Mycobacterium obuense, Mycobacterium palustre, Mycobacterium parafortuitum, Mycobacterium parascrofulaceum, Mycobacterium parmense, Mycobacterium peregrinum, Mycobacterium phlei, Mycobacterium phocaicum, Mycobacterium pinnipedii, Mycobacterium porcinum, Mycobacterium poriferae, Mycobacterium pseudoshottsii, Mycobacterium pulveris, Mycobacterium psychrotolerans, Mycobacterium pyrenivorans, Mycobacterium rhodesiae, Mycobacterium saskatchewanense, Mycobacterium scrofulaceum, Mycobacterium senegalense, Mycobacterium seoulense, Mycobacterium septicum, Mycobacterium shimoidei, Mycobacterium shottsii, Mycobacterium simiae, Mycobacterium smegmatis, Mycobacterium sphagni, Mycobacterium szulgai, Mycobacterium terrae, Mycobacterium thermoresistibile, Mycobacterium tokaiense, Mycobacterium triplex, Mycobacterium triviale, Mycobacterium tuberculosis complex (MTBC),* comprenant *Mycobacterium tuberculosis, Mycobacterium bovis, Mycobacterium bovis BCG, Mycobacterium africanum, Mycobacterium canetti, Mycobacterium caprae, Mycobacterium pinnipedii*', *Mycobacterium tusciae, Mycobacterium ulcerans, Mycobacterium vaccae, Mycobacterium vanbaalenii, Mycobacterium wolinsky et Mycobacterium xenopi.*

Egalement, l'invention, dans un mode de réalisation particulier, a pour objet l'utilisation des composés précédents pour la fabrication d'un médicament destiné au traitement ou la prévention d'infections bactériennes.

De même, l'invention concerne une méthode de traitement des infections bactériennes comprenant l'administration à des sujets, ou patients, dans le besoin des composés ou des compositions pharmaceutiques précédemment décrits, à des doses thérapeutiquement efficaces.

Le dosage de la substance active dépend particulièrement du mode d'administration, et est aisément déterminé par l'homme de métier.

Le traitement de l'invention, sans pour autant être limitatif, peut être administré par voie intraveineuse à raison de 5 à 90 µg/kg/jour.

Les composés ou les compositions pharmaceutiques de l'invention peuvent être administrées notamment par voie intraveineuse, par voie sous-cutanée, par voie systémique, par voie locale au moyen d'infiltrations ou per os.
Le traitement peut être continu ou séquentiel, c'est-à-dire au moyen d'une perfusion délivrant lesdits composés ou compositions pharmaceutiques de manière continue et éventuellement constante, ou sous forme discontinue, par une ou plusieurs prises ou injections quotidiennes, éventuellement renouvelées plusieurs jours, soit consécutifs, soit avec une latence sans traitement entre les prises.

La présente invention est illustrée par les figures et les exemples suivants. Les exemples illustrent, sans pour autant se limiter à, les modes de réalisation préférés de l'invention décrite précédemment.

Les exemples 1 à 3 illustrent les modes de synthèse des différents composés de l'invention. Les exemples 4 à 6 illustrent les propriétés des composés de l'invention sur la souche *B. suis.*

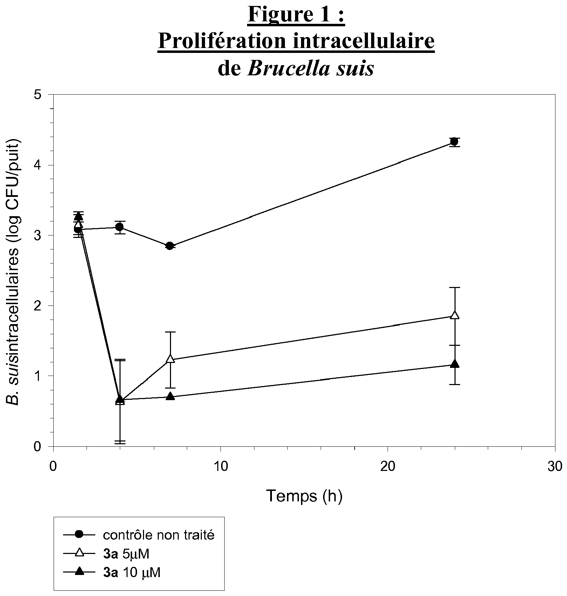

La figure 1 représente la courbe de survie intracellulaire de *Brucella suis* dans le macrophage en absence et en présence d'inhibiteur.

L'axe des abscisses (x) représente le temps exprimé en heures, l'axe des ordonnées (y) représente le logarithme du nombre de colonies de Brucella (CFU) par puits de macrophages infectés. La courbe avec les ronds pleins (●) représente le témoin, la courbe avec les triangles ouverts (Δ) représente les cellules traitées avec 5µM de composé Ib 3a, et la courbe avec les triangles noirs (▲) représente les cellules traitées avec 10µM de composé Ib 3a.

### EXEMPLES

Dans les séries de réactions ci-après, toutes les solutions ainsi que les solvants ont été dégazés par un courant d'argon avant leur utilisation. Les réactions ont été réalisées à l'échelle de 0,2g du précurseur de départ.

Les différents composés proviennent des sociétés Aldrich, Fluka ou Alfa-Aesar. Ils ont été utilisés dans les réactions sans traitement préalable particulier.

### EXEMPLE 1 : Procédure générale pour le couplage croisé de différents acides boroniques substitués avec le précurseur parabromé (1)

Le précusreur parabromé de formule générale (1) est préparé selon le procédé décrit dans Abdo et al. Bioorg. Med. Chem. 15 (2007): 4427-4433.

Dans un ballon bicol de 50 ml muni d'un réfrigérant et d'un barreau magnétique sont introduits sous flux d'argon le précurseur para bromé (1 éq.) ainsi que 5 ml de toluène.

En parallèle, dans un autre ballon de 10 ml et sous atmosphère inerte, sont dissous 3éq de l'acide boronique correspondant dans un minimum d'éthanol. Cette solution sera ensuite transvasée dans le bicol *via* une canule. Le catalyseur Pd(PPh₃)₄ (0,1 éq.) est ajouté sous flux d'argon intense, suivi de 10 éq d'une solution aqueuse de Na₂CO₃(1M). Le milieu est porté à reflux et laissé sous vive agitation pendant 12 heures. Le suivi de la réaction se fait par analyse par spectroscopie de masse car les deux produits de départ et d'arrivée présentent les mêmes rapports frontaux. La réaction achevée, le milieu réactionnel est repris avec 50 ml d'acétate d'éthyle et la phase organique est lavée à l'eau (3 fois, 15 ml) puis séchée sur sulfate de sodium anhydre. Après évaporation du solvant sous pression réduite le résidu obtenu est purifié sur colonne de gel de silice (CH₂Cl₂/ Hexane/ Acétone: 5/4/1) pour donner le produit final protégé. Ce dernier est déprotégé sous conditions acides avec une solution de HCl (4M) dans le 1,4-dioxanne. La réaction étant terminée (suivi CCM), le précipité obtenu est filtré puis lavé plusieurs fois à l'éther éthylique.

La réaction de synthèse est la suivante :

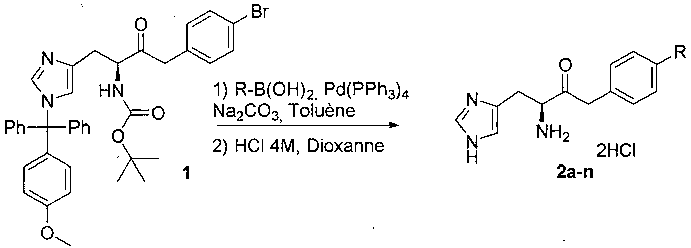

Les différents acides boroniques utilisés pour la synthèse des différents composés de l'invention sont les suivants :

| **Produit final** | **Acide boronique utilisé** |
|---|---|
| Composé Ib 2a (2a) | l'acide 4-méthylphényle boronique |
| Composé Ib 2b (2b) | l'acide 4-butylphényle boronique |
| Composé Ib 2c (2c) | l'acide 4-méthoxyphényle boronique |
| Composé Ib 2d (2d) | l'acide 4-trifluorométhylphényle boronique |
| Composé Ib 2e (2e) | l'acide 4-bromophényle boronique |
| Composé Ib 2f (2f) | l'acide 4-phénoxyphényle boronique |
| Composé Ib 2g (2g) | l'acide 4-biphényle boronique |
| Composé Ib 2h (2h) | l'acide 2-naphtyle boronique |
| Composé Ib 2i (2i) | l'acide 2-phényléthyle boronique |
| Composé Ib 2j (2j) | l'acide (E)-2-phénylvinyle boronique |
| Composé Ib 2k (2k) | l'acide (E)-2-(4-méthylphényle) vinyle boronique |
| Composé Ib 2 (2l) | l'acide (E)-2-(4-trifluorométhylphényle) vinyle boronique |
| Composé Ib 2m (2m) | l'acide (1E)-3-phényle 1-propène-1-yle boronique |
| Composé Ib 2n (2n) | l'acide (1E)-2-diphénylvinyle boronique |

### EXEMPLE 2 : Procédure générale pour le couplage des différents alcynes substitués avec le précurseur para bromé (1):

Le précusreur parabromé de formule générale (1) est préparé selon le procédé décrit dans Abdo et al. Bioorg. Med. Chem. 15 (2007): 4427-4433 (1**).**

Dans un ballon de 50 ml équipé d'un réfrigérant et d'un barreau magnétique, est dissous (1 éq.) du précurseur para bromé dans 10 ml de tétrahydrofurane (THF). A cette solution sera ajoutée l'alcyne correspondant *via* une seringue ainsi que le PPh₃ (2 éq.). Le milieu étant homogène, la base Triéthylamine (TEA) est ajoutée (3 éq.), le cocatalyseur CuI (0,2 éq.) ainsi que le catalyseur Pd(PPh₃)₄ (0,1 éq.). Le mélange est alors porté à reflux pendant 24 heures et suivi par analyse par spectroscopie de masse. Le milieu réactionnel est extrait avec 50 ml de dichlorométhane et lavé à l'eau (3 fois, 15 ml). La phase organique est séchée sur sulfate de sodium anhydre et concentrée sous pression réduite. Le résidu obtenu est directement chromatographié sur gel de silice (PhCH₃/AcOEt) : (7/3) pour donner le produit protégé pur. Ce dernier est déprotégé sous conditions acides dans une solution de HCl (4M) dans le 1,4-dioxanne. La réaction est suivie par CCM. A la fin de la réaction, le précipité obtenu est filtré et lavé plusieurs fois à l'éther éthylique.

La réaction de synthèse est la suivante :

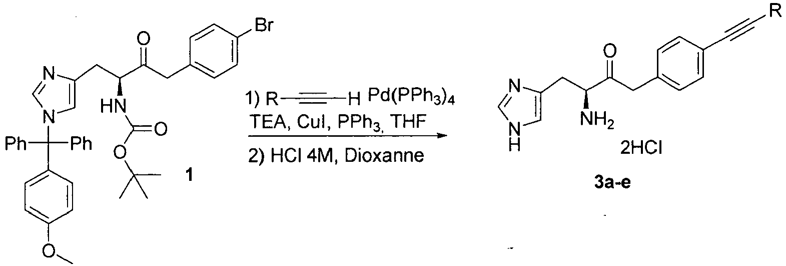

Les différents alcynes utilisés pour la synthèse des différents composés de l'invention sont les suivants :

| **Produit final** | **Alcyne utilisé** |
|---|---|
| Composé Ib 3a (3a) | le phényléthynyle |
| Composé Ib 3b (3b) | le 4 methylphenyléthynyle |
| Composé Ib 3c (3c) | le 4 tertbutylphenyléthynyle |
| Composé Ib 3d (3d) | le 4 pentylphenyléthynyle |
| Composé Ib 3e (3e) | le biphényléthynyle |

### EXEMPLE 3 : Analyses spectroscopiques et spectrométriques des différents composés obtenus selon les synthèses des exemples 1 et 2.

### Conditions générales

Les chromatographies sur couches minces (CCM) ont été réalisées sur plaques de silice en aluminium Merck 60 F354. Selon leur nature, les produits ont été révélés soit à la lumière UV (254 et 365 nm) pour les composés possédant un groupement chromophore, soit par pulvérisation d'une solution de ninhydrine dans l'éthanol pour les amines. La purification des composés par chromatographie sur colonne a été effectuée sur un gel de silice Carlo Erba (Silica Gel 60Å, granulométrie: 35-70µm). Les points de fusion non corrigés ont été déterminés en capillaire sur un appareil Büchi 530. Les spectres de masse Electro-Spray en mode positif (ESI⁺) ou en mode négatif (ESI⁻) ont été enregistrés sur un appareil LCMS Waters Micromass ZQ. Les spectres RMN du proton et du carbone 13 ont été enregistrés à température ambiante sur un Brüker DRX-400. Les déplacements chimiques sont exprimés en ppm par rapport au signal fixé à 2.5 ppm du DMSO. La multiplicité des signaux est indiquée par une ou plusieurs lettres minuscules : s (singulet), d (doublet), t (triplet), q (quadruplet), m (massif). Les constantes de couplage *J* sont exprimées en Hertz (Hz). Les solvants utilisés ont été distillés selon les méthodes décrites par D.D Perrin et W.L.F.Amarego dans Purification of Laboratory Chemicals, Permagon Press, London (1998).

Les composés obtenus et leurs analyses sont les suivants :

### Dihydrochlorhydrate de (3S)-3-amino-4-(1H-imidazol-4-yl)-1-(4'-méthylbiphényle-4-yl)butan-2-one (2a):

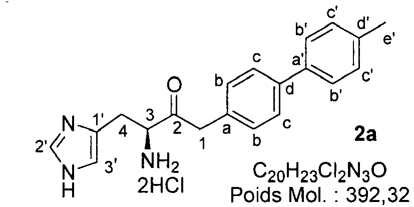

P.f. 203°C (décomposition); SM (ESI⁺/ESI⁻) : *m*/*z* 320 (M+H)⁺, 639 (2M+H)⁺ ; 318 (M-H)⁻, 354 (M+Cl)⁻, 673 (2M+Cl)⁻; ¹H RMN (400 MHz, DMSO-d₆) δ: 9,13 (d, *J*=1,1Hz, 1H, H_{2'}), 8,65 (s, 3H, NH₃⁺), 7,57 (dd, *J*=8,2Hz, *J*=17,6Hz, 4H, H_{b,c,b',c'}), 7,52 (s, 1H, H_{3'}), 7,29 (dd, J=8,1Hz, *J*=21,8Hz, 4H, H_{b,c,b',c'}), 4,73 (dd, *J*=4,1Hz, *J*=7,9Hz, 1H, H₃), 4,20 (s, 2H, H₁), 3,61 (dd, *J*=4,4Hz, *J*=15,6Hz, 1H, H₄), 3,24 (dd, *J*=8,9Hz, *J*=15,5Hz, 1H, H₄), 2,33 (s, 3H, H_{e'}); ¹³C RMN (101 MHz, DMSO-d*₆*) δ: 202,8 (1C, C₂), 138,6 (1C, C_{d'}), 136,9 (1C, C_{d,a'}), 136,6 (1C, C_{d,a'}), 134,1 (1C, C_{2'}), 132,2 (1C, Cₐ), 130,4 (2C, C_{b,c,b',c'}), 129,4 (2C, C _{b,c,b',c'}), 126,5 (1C, C_{1'}), 126,3 (2C, C_{b,c,b',c'}), 126,2 (2C, C _{b,c,b',c'}), 118,7 (1C, C_{3'}), 56,9 (1C, C₃), 44,9 (1C, C₁), 24,2 (1C, C₄), 20,6 (1C, Ce').

### Dihydrochlorhydrate de (3S)-3-amino-1-(4'-butylbiphényle-4-yl)-4-(1H-imidazol-4-yl) butan-2-one (2b):

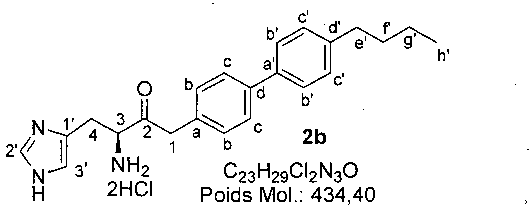

P.f. 155°C (décomposition); SM (ESI⁺/ESI⁻): *m*/*z* 362 (M+H)⁺,723 (2M+H)⁺, 360 (M-H)⁻, 396 (M+Cl)⁻, 757 (2M+Cl)⁻; ¹H RMN (400 MHz, DMSO-d*₆*) δ: 9,13 (d, *J*=1,3Hz, 1H, H_{2'}), 8,62 (m, 3H, NH₃⁺), 7,58 (dd, *J*=8,3Hz, *J*=14,9Hz, 4H, H_{b,e,b',c'}), 7,52 (d, *J*=0,9Hz, 1H, H_{3'}), 7,30 (dd, *J*=8,3Hz, *J*=18,1Hz, 4H, H_{b,c,b',c'}), 4,73 (dd, *J*=4,3Hz, *J*=9,0Hz, 1H, H₃), 4,19 (s, 2H, H₁), 3,61 (dd, *J*=4,4Hz, *J*=15,4Hz, 1H, H₄), 3,23 (dd, *J*=8,9Hz, *J*=15,5Hz, 1H, H₄), 2,61 (t, *J*=7,74Hz, 2H, H_{e'}), 1,57 (td, *J*=7,5Hz, *J*=15,2Hz, 2H, H_{f'}), 1,32 (qd, *J*=7,3Hz, *J*=14,5Hz, 2H, H_{g'}), 0,90 (t, *J*=7,3Hz, 3H,H_{h'}); ¹³C RMN (101 MHz, DMSO-d*₆*) δ: 202,8 (1C, C₂), 141,5 (1C, C_{d'}), 138,6 (1C, C_{d,a'}), 137,1 (1C, C_{d,a'}), 134,2 (1C, Cₐ), 132,2 (1C, C_{2'}), 130,4 (2C, C_{b,c,b',c'}), 128,8 (2C, C_{b,e,b',c'}), 126,5 (1C, C_{1'}), 126,3 (2C, C_{b},_{c},_{b',c'}), 126,3 (2C, C_{b,c,b',c'}), 118,3 (1C, C_{3'}), 56,9 (1C, C₃), 44,9 (1C, C₁), 34,3 (1C, C_{e'}), 33,0 (1C, C_{f'}), 24,2 (1C, C₄), 21,7 (1C, C_{g'}), 13,7 (1C, C_{h'})

### Dihydrochlorhydrate de (3S)-3-amino-4-(1H-imidazol-4-yl)-1-(4'-méthoxybiphényle-4-yl)butan-2-one (2c):

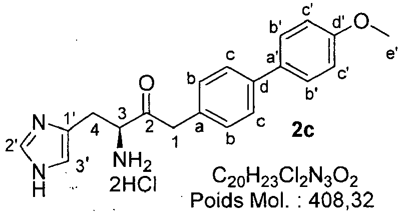

P.f. 210°C (décomposition) ; SM (ESI⁺/ESI⁻) : *m*/*z* 336 (M+H)⁺, 671 (2M+H)⁺; 334 (M-H)⁻, 370 (M+Cl)⁻, 705 (2M+Cl)⁻; ¹H RMN (400 MHz, DMSO-d*₆*) δ: 14,82 (s, 2H, NH₂⁺), 9,13 (d, *J*=1,1Hz, 1H, H_{2'}), 8,66 (s, 3H, NH₃⁺), 7,58 (dd, *J*=8,6Hz, *J*=11,4Hz, 4H, H_{b,c,b'}), 7,52 (s, 1H, H_{3'}), 7,30 (d, *J*=8,3Hz, 2H, H _{b,c,b'}), 7,01 (d, *J*=8,9Hz, 2H, H_{c'}), 4,73 (dd, 1H, *J*=4,4Hz, *J*=8,6Hz, H₃), 4,19 (s, 2H, H₁), 3,79 (s, 3H, H_{e'}), 3,61 (dd, *J*=4,3Hz, *J*=15,5Hz, 1H, H₄), 3,25 (dd, *J*=8,9Hz, *J*=15,4Hz, 1H, H₄); ¹³C RMN (101 MHz, DMSO-*d₆*) δ: 202,9 (1C, C₂), 158,8 (1C, C_{d'}), 138,3 (1C, C_{d}), 134,1 (1C, C_{2'}), 132,1 (1C, Cₐ), 131,8 (1C, C_{a'}), 130,4 (2C, C_{b,c,b'}), 127,6 (2C, C _{b,c,b'}), 126,5 (1C, C_{l'}), 125,9 (2C, C_{b,c,b'}), 118,2 (1C, C_{3'}), 114,3 (2C, C_{c'}), 56,9 (1C, C₃), 55,1 (1C, C_{e'}), 44,9 (1C, Cₗ), 24,1 (1C, C₄)

### Dihydrochlorhydrate de (3S)-3-amino-4-(1H-imidazol-4-yl)-1-[4'-(trifluorométhyle) biphényle-4-yl]butan-2-one (2d):

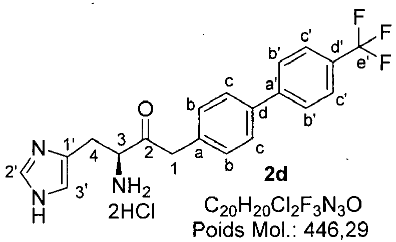

P.f. 149°C (décomposition) ; SM (ESI⁺/ESI⁻): *m*/*z* 374 (M+H)⁺, 747 (2M+H)⁺; 372 (M-H)⁻, 408 (M+Cl)⁻, 781 (2M+Cl)⁻; ¹H RMN (400 MHz, DMSO-*d₆*) δ: 14,78 (s, 2H, NH₂⁺), 9,12 (d, *J*=1,3Hz, 1H, H_{2'}), 8,64 (s, 3H, NH₃⁺), 7,90 (d, *J*=8,2Hz, 2H, H_{b,c,b'}), 7,81 (d, *J*=8,3Hz, 2H, H_{c'}), 7,71 (d, *J*=8,4Hz, 2H, H_{b,c,b'}), 7,52 (d, *J*=1,1Hz, 1H, H_{3'}), 7,39 (d, *J*=8,4Hz, 2H, H_{b,c,b'}), 4,75 (dd, *J*=4,6Hz, *J*=8,9Hz, 1H, H₃), 4,24 (s, 2H, H₁), 3,61 (dd, *J*=4,3Hz, *J*=15,5Hz, 1H, H₄), 3,24 (dd, *J*=8,9Hz, *J*=15,5Hz, 1H, H₄); ¹³C RMN (101 MHz, DMSO-*d₆*) δ: 202,7 (1C, C₂), 143,8 (1C, C_{a'}), 137,0 (1C, C_{a,d}), 134,2 (1C, C_{2'}), 133,8 (1C, C_{a,d}), 130,6 (2C, C_{b,c,b'}), 127,8 (1C, C_{d'}), 127,3 (2C, C _{b,c,b'}), 126,8 (2C, C _{b,c,b'}), 126,6 (1C, C_{1'}), 125,7 (dd, *J*=3,6Hz, *J*=7,3Hz, 2C, C_{c'}), 122,9 (1C, C_{e'}), 118,2 (1C, C_{3'}), 56,9 (1C, C₃), 44,9 (1C, C₁), 24,2 (1C, C₄)

### Dihydrochlorhydrate de (3S)-3-amino-1-(4'-bromobiphényle-4-yl)-4-(1H-imidazol-4-yl)butan-2-one (2e):

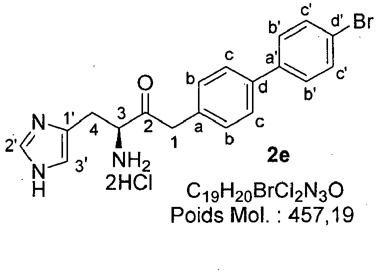

P.f. 199°C (décomposition) ; SM (ESI⁺/ESI⁻): *m*/*z* 383 (M+H)⁺, 382 (M-H)⁻, 416 (M+Cl)⁻,¹H RMN (400 MHz, DMSO-*d₆*) δ: 14,51 (s, 2H, NH₂⁺), 9,11 (d, *J*=1,0Hz, 1H, H_{2'}), 8,59 (s, 3H, NH₃⁺), 7,46 (m, 9H, H_{b,c,b',c',3'}), 4,70 (dd, *J*=4,2Hz, *J*=9,1Hz, 1H, H₃), 4,14 (s, 2H, H₁), 3,58 (dd, 1H, *J* = 15,47, 4,33 Hz, H₄), 3,20 (dd, *J*=9,0Hz, *J*=15,4Hz, 1H, H₄); ¹³C RMN (101 MHz, DMSO-*d₆*) δ: 202,8 (1C, C₂), 134,2 (1C, C_{2'}), 133,3 (1C,C_{a,d}), 130,4 (1C,C_{a,d}), 129,8 (2C, C_{b,c,b',c'}), 128,9 (1C, C_{a',d'}), 128,9 (1C, C_{a',d'}), 128,2 (2C, C_{b,c,b',c'}), 127,1 (1C, C_{1'}), 126,8 (2C, C_{b,c,b',c'}), 126,5 (2C, C_{b,c,b',c'}), 118,2 (1C, C_{3'}), 56,9 (1C, C₃), 45,2 (1C, C₁), 24,2 (1C, C₄).

### Dihydrochlorhydrate de (3S)-3-amino-4-(1H-imidazol-4-yl)-1-(4'-phénoxybiphényle-4-yl)butan-2-one (2f):

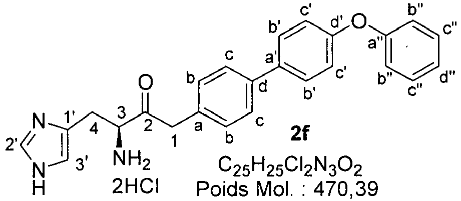

P.f. 198-200°C; SM (ESI⁺/ESI⁻): *m*/*z* 398 (M+H)⁺, 795 (2M+H)⁺; 396 (M-H)⁻, 432 (M+Cl)⁻, 829 (2M+Cl)⁻; ¹H RMN (400 MHz, DMSO-*d₆*) δ: 14,81 (s, 2H, NH₂⁺), 9,12 (d, *J*=1,08 Hz, 1H, H_{2'}), 8,66 (s, 3H, NH₃⁺), 7,68 (d, *J*=8,76 Hz, 2H, H_{b,c,b',c',b'',c''}), 7,61 (d, *J*=8,26 Hz, 2H, H_{b,c,b',c',b'',c''}), 7,52 (s, 1H, H_{3'}), 7,42 (dd, *J*=8,53, 7,47 Hz, 2H, H_{b,c,b',c',b''},_{c''}), 7,33 (d, *J*=8,26 Hz, 2H, H_{b,c,b',c',b'',c''}), 7,17 (t, *J*=7,39, 7,39 Hz, 1H, H_{d''}), 7,11-7,04 (m, 4H, H_{b,c,b',c',b''},_{c''}), 4,74 (dd, *J*=8,70, 4,53 Hz, 1H,H₃), 4,20 (s, 2H, H₁), 3,61 (dd, *J*=15,46, 4,36 Hz, 1H, H₄), 3,24 (dd, *J*=15,48, 8,86 Hz, 1H, H₄); ¹³C RMN (101 MHz, DMSO-*d₆*) δ: 202,8 (1C, C₂), 156,4 (1C, C_{d'},_{a''}), 156,3 (1C, C_{d'},_{a''}), 137,9 (1C, C_{d}), 134,9 (1C, Cₐ), 134,1 (1C, C_{2'}), 132,3 (1C, C_{a'}), 130,4-128,1 (6C, C_{b,c,b',c',b'',c''}), 126,6 (1C, C_{l'}), 126,2 (2C, C_{b,c,b',c',b'',c''}), 123,6 (1C, C_{d''}), 118,8 (4C, C_{b,c,b',c',b'',c''}), 118,2 (1C, C_{3'}), 56,9 (1C, C₃), 44,9 (1C, C₁), 24,2 (1C, C₄).

### Dihydrochlorhydrate de (3S)-3-ainino-4-(1H-imidazol-4-yl)-1-(1,1':4',1''-terphényle-4-yl)butan-2-one-(2g):

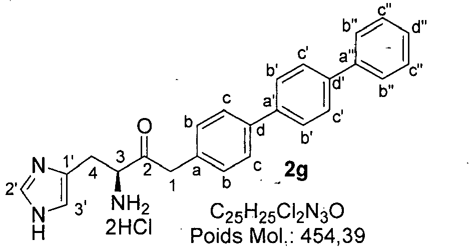

P.f. 200°C (décomposition); SM (ESI⁺/ESI⁻): *m*/*z* 382 (M+H)⁺, 763 (2M+H)⁺; 380 (M-H)⁻, 416 (M+Cl)⁻, 797 (2M+Cl)⁻; ¹H RMN (400 MHz, DMSO-*d₆*) δ: 14,71 (s, 2H, NH₂⁺), 9,12 (d, *J*=1,18 Hz, 1H, H_{2'}), 8,64 (s, 3H, NH₃⁺), 7,77 (m, 4H, H_{b,c,b',c',b",c"}), 7,75-7,65 (m, 4H, H_{,b,c,b',c',b",c"}), 7,52 (s, 1H, H_{3'}), 7,51-7,45 (m, 2H, H_{b,c,b',c',b",c"}), 7,43-7,32 (m, 3H, H_{b,c,b',c',b",c",d"}), 4,75 (dd, *J*=8,70, 4,48 Hz, 1H, H₃), 4,22 (s, 2H, H₁), 3,62 (dd, *J*=15,47, 4,33 Hz, 1H, H₄), 3,24 (dd, *J*=15,52, 8,89 Hz, 1H, H₄); ¹³C RMN (101 MHz, DMSO-*d₆*) δ: 202,8 (1C, C₂), 139,5 (1C, C_{d'},_{a"}), 139,0 (1C, C_{d'},_{a"}), 138,7 (1C, C_{d},_{a'}), 138,1 (1C, C_{d},_{a'}), 134,2 (1C, C_{2'}), 132,7 (1C, Cₐ), 130,5 (2C, C _{b,c,b',c',b",c"}), 128,9 (2C, C _{b,c,b',c',b",c"}), 127,5 (1C, Cd"), 127,1 (2C, C _{b,c,b',c',b",c"}), 127,0 (2C, C _{b,c,b',c',b",c"}), 126,6 (1C, C_{1'}), 126,5 (2C, C _{b,c,b',c',b",c"}), 126,4 (2C, C_{b,c,b',c',b",c"}), 118,3 (1C, C_{3'}), 56,9 (1C, C₃), 44,9 (1C, C₁), 24,2(1C, C₄).

### Dihydrochlorhydrate de (3S)-3-amino-4-(1H-imidazol-4-yl)-1-[4-(2-naphthyl)phényle] butan-2-one (2h):

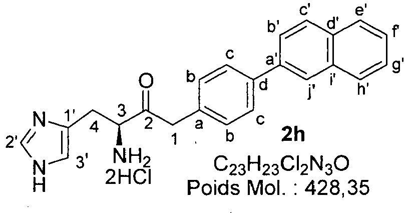

P.f. 205°C (décomposition); SM (ESI⁺/ESI⁻) : *m*/*z* 356 (M+H)⁺, 713 (2M+H)⁺; 354 (M-H)⁻, 390 (M+Cl)⁻, 745 (2M+Cl)⁻; ¹H RMN (400 MHz, DMSO-*d₆*) δ:14,86 (s, 2H, NH₂⁺), 9,14 (s, 1H, H_{2'}), 8,69 (s, 3H, NH₃⁺), 8,22 (s, 1H, H_{b',c',e',f,g',h',j'}), 8,00 (m, 2H, H_{b',c',e',f,g',h',j'}), 7,94 (m, 1H, H_{b',c',e',f,g',h',j'}),7,85 (dd, *J*=1,6Hz, *J*=8,6Hz, 1H, H_{b',c',e',f',g',h',j'}), 7,79 (d, *J*=8,2Hz, 2H, H_{c}),7,53 (m, 3H, H_{b',c',e',f,g',h',j',3'}), 7,40 (d, *J*=8,2Hz, 2H, H_{b}), 4,76 (dd, *J*=4,7Hz, *J*=8,7Hz, 1H, H₃), 4,25 (s, 2H, H₁), 3,63 (dd, *J*=4,2Hz, *J*=15,4Hz, 1H, H₄), 3,27 (dd, *J*=9,1HZ, *J*=15,6Hz, 1H, H₄); ¹³C RMN (101 MHz, DMSO-*d₆*) δ: 202,8 (1C, C₂), 138,4 (1C, C_{d},_{a'}), 137,1 (1C, C_{d},_{a'}), 134,1 (1C, C_{2'}), 133,3 (1C, Cₐ), 132,8 (1C, C_{d'},_{i'}), 132,1 (1C, C_{d'},_{i'}), 130,5 (2C, C_{b}), 128,4-127,4 (3C, C_{b',c',e',f,g',h',j'}), 126,8 (2C, C_{c}), 126,6-126,0 (3C, C_{b',c',e',f,g',h',j',l'}), 125,0-124,9 (2C, C_{b',c',e',f,g',h',j'}), 118,3 (1C, C_{3'}), 56,9 (1C, C₃), 44,9 (1C, C₁), 24,2 (1C, C₄).

### Dihydrochlorhydrate de (3S)-3-amino-4-(1H-imidazol-4-yl)-1-[4-(2-phényléthylé)phényle] butan-2-one (2i):

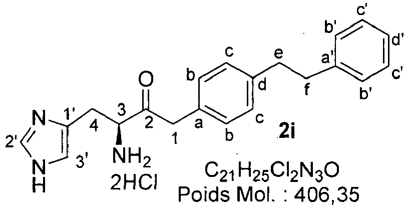

P.f. 168-170°C; SM (ESI⁺/ESI⁻): *m*/*z* 334 (M+H)⁺, 667 (2M+H)⁺; 332 (M-H)⁻, 368 (M+Cl)⁻, 701 (2M+Cl)⁻; ¹H RMN (400 MHz, DMSO-*d₆*) δ: 9,11 (s, 1H, H_{2'}), 8,63 (s, 3H, NH₃⁺), 7,50 (s, 1H, H_{3'}), 7,31-7,20 (m, 4H, H_{b,c,b',c'}), 7,21-7,08 (m, 5H, H_{b,c,b',c',d'}), 4,69 (dd, *J*=8,03, 4,09 Hz, 1H, H₃), 4,10 (s, 2H, H₁), 3,57 (dd, *J* = 15,41, 4,14 Hz, 1H, H₄), 3,21 (dd, *J* = 15,39, 8,89 Hz, 1H, H₄), 2,86 (s, 4H,H_{e,f}); ¹³C RMN (101 MHz, DMSO-*d₆*) δ: 202,9 (1C, C₂), 141,4 (1C, C_{a'},_{d}), 140,0 (1C, C_{a'},_{d}), 134,1 (1C, C_{2'}), 130,7 (1C, Cₐ), 129,7-128,1 (8C, C _{b,c,b',c'}), 126,5 (1C, C_{1'}), 125,7 (1C, C_{d'}), 118,2 (1C, C_{3'}), 56,9 (1C, C₃), 44,9 (1C, C₁), 36,9 (1C, Cₑ,_{f}), 36,6 (1C, Cₑ,_{f}), 24,1 (1C, C₄).

### Dihydrochlorhydrate de (3S)-3-amino-4-(1H-imidazol-4-yl)-1-{4-[(E)-2-phénylvinyle] phényle}butan-2-one (2j):

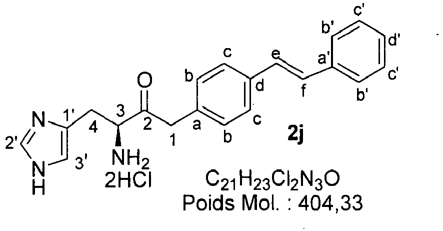

P.f. 195°C (décomposition); SM (ESI⁺/ESI⁻): *m*/*z* 332 (M+H)⁺, 663(2M+H)⁺;330 (M-H)⁻, 366 (M+Cl)⁻, 697 (2M+Cl)⁻; ¹H RMN (400 MHz, DMSO-*d₆*) δ: 14,58 (s, 2H, NH₂⁺), 9,12 (d, *J*=1,4Hz, 1H, H_{2'}), 8,61 (s, 3H, NH₃⁺), 7,59 (m, 3H, H_{b,c,b',c',d'}), 7,51 (d, *J*=1,2Hz, 1H, H_{3'}), 7,27 (m, 8H, H_{b,c,b',c',d'}), 4,72 (dd, *J*=4,5Hz, *J*=8,6Hz, 1H, H₃), 4,16 (s, 2H, H₁), 3,59 (dd, *J*=4,1Hz, *J*=15,3Hz, 1H, H₄), 3,22 (dd, *J*=8,9Hz, *J*=15,5Hz, 1H, H₄); ¹³C RMN (101 MHz, DMSO- *d₆*) δ: 202,8 (1C, C₂), 136,9 (1C, C_{d,a'}), 135,6 (1C, C_{d,a'}), 134,2 (1C, C_{2'}), 132,7 (1C, Cₐ), 130,2 (2C, C_{b,c,b',c'}), 128,6 (2C, C_{b,c,b',c'}), 128,2 (1C, C_{e,,f,,d'}), 127,9 (1C, C_{e,,f,,d'}), 127,6 (1C, C_{e,,f,,d'} ), 126,5 (1C, C_{1'}), 126,4 (2C, C_{b,c,b',c'}), 126,3 (2C, C_{b,c,b',c'}), 118,2 (1C, C_{3'}), 56,9 (1C, C₃), 45,0 (1C, C₁), 24,2 (1C, C₄).

### Dihydrochlorhydrate de (3S)-3-amino-4-(1H-imidazol-4-yl)-1-{4-[(E)-2-(4-méthylphényle) vinyle]phényle}butan-2-one (2k):

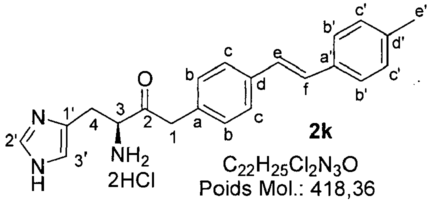

P.f. 200°C (décomposition); SM (ESI⁺/ESI⁻): *m*/*z* 346 (M+H)⁺, 691 (2M+H)⁺; 344 (M-H)⁻, 380 (M+Cl)⁻, 725 (2M+Cl)⁻; ¹H RMN (400 MHz, DMSO-*d₆*) δ: 14,56 (s, 2H, NH₂⁺), 9,12 (d, *J*=1,3Hz, 1H, H_{2'}), 8,61 (s, 3H, NH₃⁺), 7,54 (d, *J*=8,3Hz, 2H, H_{b,c,b',c'}), 7,51 (d, *J*=0,8Hz, 1H, H_{3'}), 7,49 (d, *J*=8,2Hz, 2H, H_{b,c,b',c'}), 7,24 (d, *J*=8,3Hz, 2H, H_{b,c,b',c'}), 7,18 (m, 4H, H_{b,c,e,f,b',c'}), 4,71 (dd, *J*=4,5Hz, *J*=8,0Hz, 1H, H₃), 4,15 (s, 2H, H₁), 3,59 (dd, *J*=4,5Hz, *J*=15,5Hz, 1H, H₄), 3,22 (dd, *J*=9,0Hz, *J*=15,5Hz, 1H, H₄), 2,30 (s, 3H, H_{e'}); ¹³C RMN (101 MHz, DMSO- *d₆*) δ: 202,8 (1C, C₂), 136,9 (2C, C_{d,a'}), 135,8 (1C, C_{a,d'}), 134,2 (1C, C_{2'}), 132,5 (1C, C_{a,d'}), 130,2 (2C, C_{b,c,b',c'}), 129,2 (2C, C_{b,c,b',c'}), 128,1 (1C, C_{e,f}), 126,9 (1C, C_{e,f}), 126,5 (1C, C_{1'}), 126,3 (2C, C_{b,c,b',c'}), 126,2 (2C, C_{b,c,b',c'}), 118,2 (1C, C_{3'}), 56,9 (1C, C₃), 45,0 (1C, C₁), 24,2 (1C, C₄), 20,8 (1 C, C_{e'}).

### Dihydrochlorhydrate de (3S)-3-amino-4-(1H-imidazol-4-yl)-1-(4-{(E)-2-[4-(trifluoro méthyl)phényle]vinyle}phényle) butan-2-one (21):

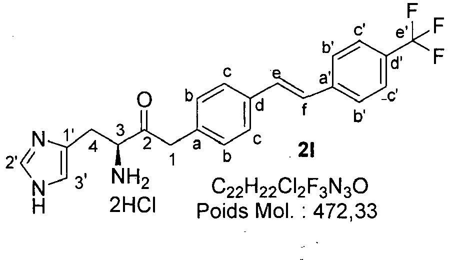

P.f. 186°C (décomposition); SM (ESI⁺/ESI⁻): *m*/*z* 399 (M+H)⁺, 799 (2M+H)⁺; 398 (M-H)⁻, 434 (M+Cl)⁻, 833 (2M+Cl)⁻; ¹H RMN (400 MHz, DMSO-*d₆*) δ: 14,56 (s, 2H, NH₂⁺), 9,13 (s, 1H, H_{2'}), 8,62 (s, 3H, NH₃⁺), 7,82 (d, *J*=8,1Hz, 2H, H_{b,c,b'}), 7,72 (d, *J*=8,6Hz, 2H, H_{b,c,b'}), 7,62 (d, *J*=8,2Hz, 2H, H_{b,c,b'}), 7,52 (d, *J*=0,6Hz, 1H, H_{3'}), 7,43 (d, *J*=16,6Hz, 1H, H_{e,f}), 7,35 (d, *J*=16,4Hz, 1H, H_{e,f}), 7,28 (d, *J*=8,1Hz, 2H, H_{c'}), 4,72 (s 1H, H₃), 4,18 (s, 2H, H₁), 3,60 (dd, *J*=4,6Hz, *J*=15,8Hz, 1H, H₄), 3,22 (dd, *J*=9,2Hz, *J*=15,5Hz, 1H, H₄); ¹³C RMN (101 MHz, DMSO-*d₆*) δ: 202,7 (1C, C₂), 141,1 (1C, C_{d,a'}), 135,1 (1C, C_{d,a'}), 134,1 (1C, C_{2'}), 133,4 (1C, Cₐ), 130,9 (1C, C_{e,f}), 130,2 (2C, C_{b,c,b'}), 127,2 (1C, C_{d'}), 126,9 (2C, C_{b,c,b'}), 126,7 (2C, C_{b,c,b'}), 126,6 (1C, C_{1'}), 126,5 (1C, C_{e,f}), 125,5 (m, 2C, C_{c'}), 122,9 (1C, C_{e'}), 118,2 (1C, C_{3'}), 56,9 (1C, C3), 45,0 (1C, C₁), 24,1 (1C, C₄).

### Dihydrochlorhydrate de (3S)-3-amino-4-(1H-imidazol-4-yl)-1-{4-[(1E)-3-phénylprop-1-èn-1-yl]phényle}butan-2-one (2m):

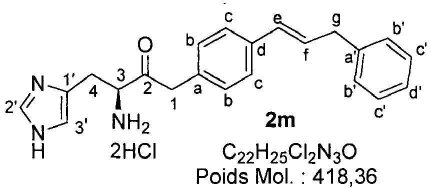

P.f. 178-180°C; SM (ESI⁺/ESI⁻): *m*/*z* 346 (M+H)⁺, 691 (2M+H)⁺; 344 (M-H)⁻, 380 (M+Cl)⁻, 725 (2M+Cl)⁻; ¹H RMN (400 MHz, DMSO-*d₆*) δ: 14,71 (s, 2H, NH₂⁺), 9,11 (d, *J*=1,2Hz, 1H, H_{2'}), 8,61 (s, 3H, NH₃⁺), 7,50 (s, 1H, H_{3'}), 7,27 (m, 9H, H_{b,c,b',c',d'}), 6,47 (d, 1H, *J*=15,8Hz,Hₑ), 6,41 (dd, 1H, *J*=16,5Hz,*J*=6,1Hz, H_{f}), 4,69 (dd, *J*=4,8Hz, *J*=8,7Hz, 1H,H₃), 4,12 (s, 2H, H₁), 3,57 (dd, *J*=4,3Hz, *J*=15,6Hz, 1H, H₄), 3,52 (d, *J*=5,6Hz, 2H, Hg), 3,21 (dd, *J*=8,9Hz, *J*=15,4Hz, 1H, H₄); ¹³C RMN (101 MHz, DMSO-*d₆*) δ: 202,8 (1C, C₂), 139,9 (1C, C_{d,a'}), 135,6 (1C, C_{d,a'}), 134,1 (1C, C_{2'}), 132,1 (1C, Cₐ), 130,1 (1C, Cₑ), 130,0 (2C, C_{b,c,b',c'}), 129,2 (1C, C_{f}), 128,4 (2C, C_{b,c,b',c'}), 128,4 (2C, C_{b,c,b',c'}), 126,5 (1C, C_{1'}), 125,9 (1C, C_{d'}), 125,8 (2C, C_{b,c,b',c'}), 118,2 (1C, C_{3'}), 56,9 (1C, C₃), 44,9 (1C, C₁), 38,5 (1C, Cg), 24,1 (1C, C₄).

### Dihydrochlorhydrate de (3S)-3-amino-1-{4-[(E)-2-biphényle-4-ylvinyle]phényle}-4-(1H-imidazol-4-yl)butan-2-one (2n):

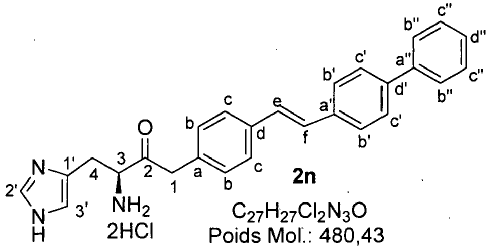

P.f. 200°C (décomposition); SM (ESI⁺/ESI⁻): *m*/*z* 408 (M+H)⁺, 815 (2M+H)⁺; 406 (M-H)⁻, 442 (M+Cl)⁻, 849 (2M+Cl)⁻; ¹H RMN (400 MHz, DMSO-*d₆*) δ: 14,54 (s, 2H, NH₂⁺), 9,12 (d, *J*=1,3Hz, 1H, H_{2'}), 8,59 (s, 3H, NH₃⁺), 7,70 (m, 6H, H_{b,c,b',c',b",c"}), 7,60 (d, *J*=8,4Hz, 2H, H_{b,c,b',c',b",c"}), 7,51 (d, *J*=1,0Hz, 1H, H_{3'}), 7,47 (t, *J*=7,6Hz, 2H, H_{b,c,b',c',b",c"}), 7,37 (t, *J*=1,2Hz, 1H, H_{d"}), 7,31 (m, 2H, H_{b,c,b',c',b",c"}), 7,27 (d, *J*=8,3Hz, 2H, H_{b,c,b',c',b",c",d"}), 4,72 (dd, *J*=4,3Hz, *J*=8,3Hz, 1H, H₃), 4,17 (s, 2H, H₁), 3,59 (dd, *J*=4,3Hz, *J*=15,3Hz, 1H, H₄), 3,22 (dd, *J*=8,9Hz, *J*=15,5Hz, 1H, H₄); ¹³C RMN (101 MHz, DMSO-*d₆*) δ: 202,8 (1C, C₂), 139,6 (1C, C_{d,a',d',a"}), 139,1 (1C, C_{d,a',d',a"}), 136,2 (1C, C_{d,a',d',a"}), 135,7 (1C, C_{d,a',d',a"}), 134,3 (1C, C_{2'}), 132,8 (1C, Cₐ), 130,3-126,4 (16C, C_{b,c,b',c',b",c"d",e,f,1'}), 118,3 (1C, C_{3'}), 56,9 (1C, C₃), 45,1 (1C, C₁), 24,3 (1C, C₄).

### Dihydrochlorhydrate de (3S)-3-amino-4-(1H-imidazol-4-yl)-1-[4-(phényléthynyle)phényle] butan-2-one (3a):

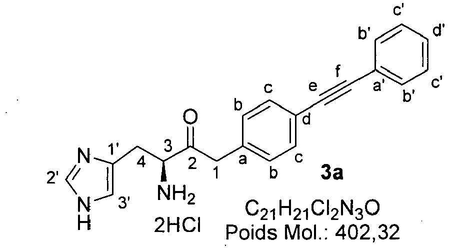

P.f. 194°C (décomposition); SM (ESI⁺/ESI⁻): *m*/*z* 330 (M+H)⁺, 659 (2M+H)⁺; 328 (M-H)⁻, 364 (M+Cl)⁻, 693 (2M+Cl)⁻; ¹H RMN (400 MHz, DMSO- *d₆*) δ: 14,69 (s, 2H, NH₂⁺), 9,11 (d, *J*=1,1Hz, 1H, H_{2'}), 8,63 (s, 3H, NH₃⁺), 7,53 (m, 5H, H_{b,c,b',c',d',3'}), 7,43 (m, 2H, H_{b,c,b',c',d'}), 7,27 (m, 2H, H_{b,c,b',c',d'}), 4,72 (dd, *J*=4,2Hz, *J*=8,5Hz, 1H, H₃), 4,23 (s, 2H, H₁), 3,59 (dd, *J*=4,4Hz, *J*=15,4Hz, 1H, H₄), 3,23 (dd, *J*=8,9Hz, *J*=15,8Hz, 1H, H₄) ;¹³C-RMN (101 MHz, DMSO- *d₆*) δ: 202,5 (1C, C₂), 134,2 (1C, C_{2'}), 134,2 (1C, Cₐ), 132,1 (1C, C_{b,c,b',c',d'}), 131,3 (2C, C_{b,c,b',c',d'}), 131,2 (2C, C_{b,c,b',c',d'}), 130,3 (2C, C_{b,c,b',c',d}'), 128,7 (2C, C_{b,c,b',c',d'}), 126,5 (1C, C_{1'}), 122,2 (1C, C_{d,a'}), 120,7 (1C, C_{d,a'}), 118,2 (1C, C_{3'}), 89,2 (1C, C_{e,f}), 89,1 (1C, C_{e,f}), 56,9 (1C, C₃), 45,1 (1C, C₁), 24,1 (1C, C₄).

### Dihydrochlorhydrate de (3S)-3-amino-4-(1H-imidazol-4-yl)-1-{4-[(4-méthylphényle) éthynyle]phényle}butan-2-one (3b):

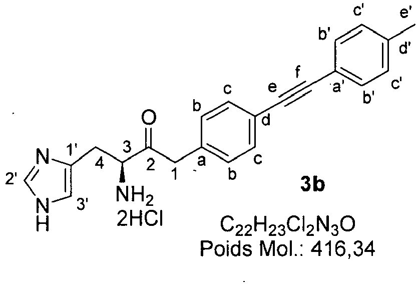

P.f. 181°C (décomposition); SM (ESI⁺/ESI⁻): *mlz* 344 (M+H)⁺, 687 (2M+H)⁺; 342 (M-H)⁻, 378 (M+Cl)⁻, 722 (2M+Cl)⁻; ¹H RMN (400 MHz, DMSO-*d₆*) δ (ppm): 14,53 (s, 2H, NH₂⁺), 9,13 (s, 1H, H_{2'}), 8,64 (s, 3H, NH₃⁺), 7,46 (m, 5H, H_{b,c,b',c',d',3'}), 7,26 (d, 4H, H_{b,c,b',c',d'}), 4,72 (s large, 1H, H₃), 4,22 (s, 2H, H₁), 3,59 (dd, *J*=4,5Hz, *J*=15,4Hz, 1H, H₄), 3,23 (dd, *J*=8,9Hz, *J*=15,7Hz, 1H, H₄), 2,33 (s, 3H, H_{e'}) ; ¹³C-RMN (101 MHz, DMSO-*d₆*) δ (ppm): 202,6 (1C, C₂), 138,5 (1C, C_{d'}), 134,1 (1C, C_{2'}), 134,0 (1C, Cₐ), 131,2 (2C, C_{b,c,b',c'}), 131,1 (2C, C_{b,c,b',c'}), 130,3 (2C, C_{b,c,b',c'}), 129,3 (2C, C_{b,c,b',c'}), 126,5 (1C, C_{1'}), 120,9 (1C, C_{d},_{a'}), 119,2 (1C, C_{d},_{a'}), 118,3 (1C, C_{3'}), 89,4 (1C, C_{e,f}), 88,5 (1C, C_{e,f}), 56,9 (1C, C₃), 45,1 (1C, C₁), 24,1 (1C, C₄), 20,9 (1C, C_{e'}).

### Dihydrochlorhydrate de (3S)-3-amino-1-{4-[(4-tert-butylphényle)éthynyl]phényle}-4-(1H-imidazol-4-yl)butan-2-one (3c):

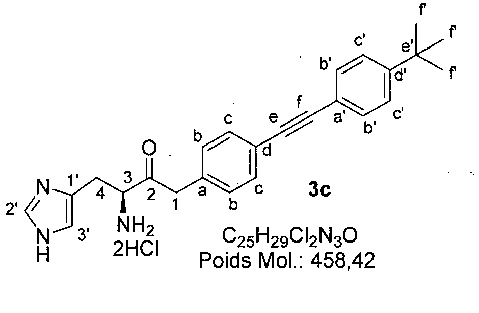

P,f, 197°C (décomposition); SM (ESI⁺/ESI⁻): *mlz* 386 (M+H)⁺, 771 (2M+H)⁺; 384 (M-H)⁻, 420 (M+Cl)⁻, 805 (2M+Cl)⁻; ¹H RMN (400 MHz, DMSO-*d₆*) δ : 14,49 (s, 2H, NH₂⁺), 9,11 (d, *J*=1,3Hz, 1H, H_{2'}), 8,59 (s, 3H, NH₃⁺), 7,48 (m, 7H, H_{c,b',c',3'}), 7,29 (d, *J*=8,4Hz, 2H, H_{b}), 4,72 (dd, *J*=5,0Hz, *J*=8,8Hz, 1H, H₃), 4,21 (s, 2H, H₁), 3,58 (dd, *J*=4,5Hz, *J*=15,5Hz, 1H, H₄), 3,21 (dd, *J*=8,9Hz, *J*=15,5Hz, 1H, H₄), 1,29 (s, 9H, H_{f'}); ¹³C-RMN (101 MHz, DMSO-*d₆*) δ: 202,5 (1C, C₂), 151,5 (1C, C_{d'}), 134,3 (1C, C_{2'}), 134,0 (1C, Cₐ), 131,1 (2C, C_{c,b',c'}), 131,0 (2C, C_{c,b',c'}), 130,3 (2C, C_{b}), 126,5 (1C, C_{1'}), 125,5 (2C, C_{c,b',c'}), 120,9 (1C, C_{d,a'}), 119,3 (1C, C_{d,a'}), 118,3 (1C, C_{3'}), 89,4 (1C, Cₑ,_{f}), 88,6 (1C, Cₑ,_{f}), 56,9 (1C, C₃), 45,1 (1C, C₁), 34,5 (1C, C_{e'}), 30,8 (3C, C_{f'}), 24,2 (1C, C₄).

### Dihydrochlorhydrate de (3S)-3-amino-4-(1H-imidazol-4-yl)-1-{4-[(4-pentylphényle) éthynyle]phényle}butan-2-one (3d):

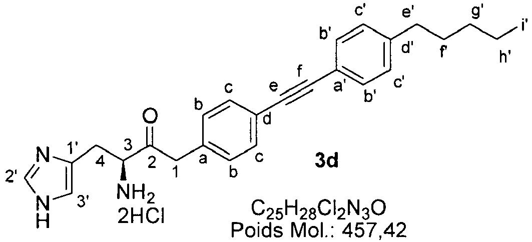

P.f. 184°C (décomposition); SM (ESI⁺/ESI⁻): *m*/*z* 400 (M+H)⁺, 799 (2M+H)⁺; 398 (M-H)⁻, 434 (M+Cl)⁻, 833 (2M+Cl)⁻; ¹H RMN (400 MHz, DMSO-*d₆*) δ: 14,55 (s, 2H, NH₂⁺), 9,12 (d, *J*=1,3Hz, 1H, H_{2'}), 8,62 (s, 3H, NH₃⁺), 7,50 (m, 5H, H_{b,c,b',c',3'}), 7,26 (m, 4H, H_{b,c,b',c'}), 4,72 (dd, *J*=4,3Hz, *J*=8,2Hz, 1H, H₃), 4,22 (s, 2H, H₁), 3,59 (dd, *J*=4,5Hz, *J*=15,5Hz, 1H, H₄), 3,22 (dd, 1H, *J*=9,0Hz, *J*=15,5Hz, H₄), 2,59 (t, *J*=7,5Hz, 2H, H_{e'}), 1,57 (td, *J*=7,5Hz, *J*=14,9Hz, 2H, H_{f'}), 1,27 (m, 4H, H_{g'},_{h'}), 0,85 (t, *J*=7,0Hz, 3H, H_{i'}); ¹³C RMN (101 MHz, DMSO-*d₆*) δ: 202,5 (1C, C₂), 143,3 (1C, C_{d'}), 134,2 (1C, C_{2'}), 134,0 (1C, Cₐ), 131,2 (2C, C_{b,b',c,c'}), 131,1 (2C, C_{b,b',c,c'}), 130,3 (2C, C_{b,b',c,c'}), 128,6 (2C, C_{b,b',c,c'}), 126,5 (1C, C_{1'}), 120,9 (1C, C_{d},ₐ), 119,4 (1C, C_{d},ₐ), 118,3 (1C, C_{3'}), 89,4 (1C, Cₑ,_{f}), 88,5 (1C, Cₑ,_{f}), 56,9 (1C, C₃), 45,1 (1C, C₁), 34,9 (1C, C_{e'}), 30,759 (1C, C_{g'}), 30,3 (1C, C_{f'}), 24,1 (1C, C₄), 21,8 (1C, C_{h'}), 13,8 (1C, C_{i'})

### Dihydrochlorhydrate de (3S)-3-amino-l-[4-(biphényle-4-yléthynyle)phényle]-4-(1H-imida zol-4-yl)butan-2-one (3e):

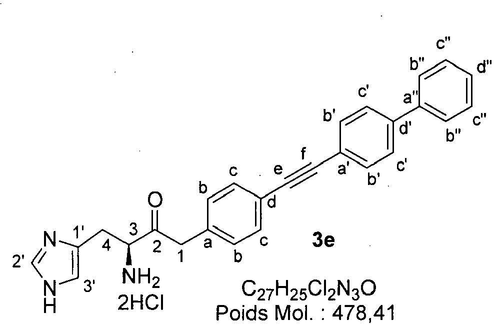

P.f. 196°C (décomposition); SM (ESI⁺/ESI⁻): *m*/*z* 406 (M+H)⁺, 811 (2M+H)⁺; 404 (M-H)⁻, 440 (M+Cl)⁻,_845 (2M+Cl)⁻,_¹H RMN (400 MHz, DMSO-d₆) δ:14,56 (s, 2H, NH₂⁺), 9,12 (d, *J*=1,3Hz, 1H, H_{2'}), 8,61 (s, 3H, NH₃⁺), 7,73 (m, 4H, H_{b,c,b',c',b",c"}), 7,64 (m, 2H, H_{b,c,b',c',b",c"}), 7,56 (m, 2H, H_{b,c,b',c',b",c"}), 7,52 (d, *J*=1,0Hz, 1H, H_{3'}), 7,49 (m, 2H, H_{b,c,b',c',b",c"}), 7,40 (m, 1H, H_{d"}), 7,31 (m, 2H, H_{b,c,b',c',b",c"}), 4,73 (dd, *J*=4,3Hz, *J*=8,3Hz, 1H, H₃), 4,23 (s, 2H, H₁), 3,60 (dd, *J*=4,3Hz, *J*=15,7Hz, 1H, H₄), 3,22 (dd, *J*=8,9Hz, *J*=15,6Hz, 1H, H₄); ¹³C RMN (101 MHz, DMSO-*d₆*) δ: 202,5 (1C, C₂), 140,2 (1C, C_{d'},_{a"}), 139,0 (1C, C_{d'},_{a"}), 134,2 (1C, Ca), 134,2 (1C, C_{2'}), 131,9 (2C, C_{b,c,b',c',b",c"}), 131,2 (2C, C_{b,c,b"c',b",c"}), 130,3 (2C, C_{b,c,b',c',b",c"}) , 129,0 (2C, C_{b,c,b',c',b",c"}), 127,9 (1C, C_{d"}), 126,9 (2C, C_{b,c,b',c',b",c"}), 126,6 (2C, C_{b,c,b',c',b",c"}), 126,5 (1C, C_{1'}), 121,2 (1C, C_{d},_{a'}), 120,7 (1C, C_{d},_{a'}), 118,3 (1C, C_{3'}), 89,9 (1C, Cₑ,_{f}), 89,2 (1C,Cₑ,_{f}), 57,0 (1C, C₃), 45,1 (1C, C₁), 24,2 (1C, C₄)

### Exemple 4 : Mesure de l'effet inhibiteur des différents composés de l'invention sur l'histidinol déshydrogénase de B. suis purifiée.

L'activité de HDH vis-à-vis des différents composés a été mesurée en suivant la réduction de NAD⁺ en NADH directement à 340 nm (ε_{M}=6200 M⁻¹cm⁻¹) comme décrit dans l'art antérieur (*Loper, J. C.; Adams, E. J. Biol. Chem. 240 (1965): 788-795*). L'activité enzymatique a été étudiée à 30°C en présence de 0,5mM d'histidinol, 5mM de NAD⁺ et 0.5mM de MnCl₂ dans un tampon glycinate de sodium 50mM pH=9,2.
Pour les études cinétiques, les expériences ont été réalisées avec du glycinate de sodium 150mM (pH=9,2) et du NAD⁺ 2mM.

Le Kₘ pour le substrat a été déterminé en variant la concentration de l'histidinol de 10 à 50µM. L'activité (1U) est définie comme la quantité de HDH produisant 1µM de NADH par minute dans la réaction.

Pour déterminer les CI₅₀ des différents inhibiteurs, ces derniers ont été ajoutés à différentes concentrations allant de 1 à 200nM et pré-incubés pendant 5 minutes à 30°C avec la solution d'enzyme avant l'initiation de la réaction.

La concentration en enzyme dans le système est de 4. 10⁻¹¹M.

Les résultats obtenus (CI₅₀) pour les différents inhibiteurs sont présentés dans le tableau 1 suivant :

**Tableau 1 : CI₅₀ des différents composés vis-à-vis de l'activité catalytique de l'histidinol déshydrogénase de B. suis (nd= non déterminé)**

| **Composés** | ***B. suis* HDH CI₅₀ (nM)** | **Composés** | ***B. suis* HDH CI₅₀ (nM)** |
|---|---|---|---|
| **2a** | 30 | **2k** | 40 |
| **2b** | 40 | **2l** | 65 |
| **2c** | 20 | **2m** | 30 |
| **2d** | 30 | **2n** | nd |
| **2e** | nd | **3a** | 3 |
| **2f** | 70 | **3b** | 40 |
| **2g** | nd | **3c** | 65 |
| **2h** | 30 | **3d** | 8.5 |
| **2i** | 13 | **3e** | nd |
| **2j** | 25 | | |

Le tableau 1 montre que tous les composés de l'invention testés ont une affinité élévée pour l'HDH (CI₅₀ comprise entre 70 et 3 nM). Le composé 3a possède une affinité importante pour l'HDH.

Comparé aux composés publiés dans Abdo *et al* [*Abdo et al. Bioorg. Med. Chem. 15 (2007): 4427-4433*], les composés selon l'invention présentent une activité inhibitrice comparable de l'ordre du nanomolaire sur l'HDH purifiée.

Des résultats similaires sont obtenus sur l' HDH de Mycobacterium.

### Exemple 5 : Effets des inhibiteurs de la HDH de B. suis sur la croissance de B. suis en milieu minimum

Une culture en phase stationnaire de *B. suis* est réalisée sur 24 h dans du bouillon TS (tryptic soja) à partir d'une strie de la bactérie sur gélose. Le culot bactérien est récupéré par centrifugation, puis lavé (PBS) et resuspendu dans du milieu minimum [Gerhardt et al, J Bacteriol 59 (1950): 777-782] dans le volume de la culture d'origine. 30 microlitres de cette suspension bactérienne sont utilisés pour ensemencer 3ml de milieu minimum.Typiquement, les inhibiteurs à étudier sont ensuite rajoutés à des concentrations de 10, 50 ou 100 micromolaires. Une culture contrôle ne contient pas d'inhibiteur.

Les cultures sont agitées à 37°C et la croissance est suivie par mesure de DO (600 nm) à 48, 72 et 96 h.

Les résultats correspondant au pourcentage d'inhibition de la croissance bactérienne en milieu minimum sont présentés dans le tableau 2 suivant :

**Tableau 2 : inhibition de la croissance bactérienne en milieu liquide des bactéries B. suis en présence de 100µM ou 50µM.**

| ***Composés*** | **Inhibition de *B. suis* HDH CI₅₀** | **Effets sur la croissance de *B. suis* (%) 7 jours en milieu minimum** | |
|---|---|---|---|
| | **(nM)** | **100µM** | **50µM** |
| **2a** | 30 | 40 | 30 |
| **2b** | 40 | 60 | 50 |
| **2c** | 20 | 60 | 30 |
| **2d** | 30 | 90 | 70 |
| **2f** | 70 | 90 | 70 |
| **2h** | 30 | 100 | 100 |
| **2k** | 40 | 100 | 100 |
| **3a** | 3 | 100 | 100 |
| **3c** | 65 | 30 | 30 |
| **3d** | 8.5 | 30 | 30 |

Les résultats indiquent que les composés 2h, 2k et particulièrement 3a, inhibent complètement la croissance des bactéries en milieu liquide, dès une concentration de 50µM.

Ces résultats montrent également que les inhibiteurs sont capables d'atteindre leur cible dans le pathogène intact et vivant, confirmant ainsi leur capacité de traverser les membranes procaryotes.

### Exemple 6 : Effets des inhibiteurs de la HDH de B. suis sur la croissance de B. suis dans le macrophage

Les cellules différenciées de la lignée macrophagique transformée humaine THP-1 sont ensemencées à une concentration de 500000 cell./ml/puits dans des plaques 24-puits et incubées pendant 24 h à 37°C/5% CO₂ en milieu RPMI/10% SVF. L'infection des cellules est réalisée à une multiplicité d'infection (MOI) de 20. Pour cela, la quantité appropriée d'une culture stationnaire de *B. suis* (10¹⁰ bactéries/ml) est reprise dans 100 microlitres de RPMI par puits de cellules et rajoutée sur les cellules THP-1. Après incubation pendant 45 min, les cellules sont lavées 2 fois avec du PBS et incubées avec 1ml de RPMI/10% SVF par puits contenant de la gentamicine (30 microgrammes/ml). Après 1h d'incubation, les cellules de la première série de puits sont lavées 1 fois au PBS, lysées avec du triton X-100 (0,2%) et étalées sur gélose TS. Le nombre de bactéries intracellulaires est dénombré après incubation à 37°C pendant 3 jours. Les puits restants sont départagés en puits non traités (contrôle) et en puits traités avec l'inhibiteur d'intérêt (typiquement à 10 et 25 µM en RPMI). Les lyses et étalements (voir ci-dessus) sont effectués à 4h, 7h et 24h après l'infection. Les expériences sont réalisées en triplicats.

Les résultats de prolifération de B. suis intracellulaire traités par le composé 3a sont présentés dans la figure 1.

Les résultats expérimentaux montrent que le composé 3a est capable de pénétrer dans les cellules THP-1 infectées par *B. suis.* Egalement, les résultats montrent que le composé 3a inhibe efficacement (environ 1000 fois) la prolifération intracellulaires des bactéries lors quelles sont dans le macrophage. Ce composé est donc actif non seulement sur les bactéries isolés en milieu minimum, mais également sur le pathogène à l'état intracellulaire. L'inhibiteur facilitera donc par la suite l'élimination du pathogène par le système immunitaire de l'hôte.

## Revendications

1. Composés de formule générale suivante (Ia): 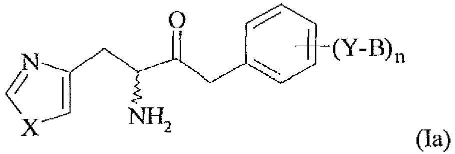 où X représente un N
**caractérisés en ce que**
- le groupe phényle est substitué par n groupement(s) Y-B, n variant de 1 à 5, ledit groupement Y-B étant tel que :
- Y représente un groupe alkylène linéaire ou ramifié, saturé ou insaturé, comprenant de 1 à 6 atomes de carbone, de préférence de 2 à 4 atomes de carbone, et
- B représente un groupe choisi parmi :
- un groupe aryle ou hétéroaryle, notamment un groupe phényle e ou naphtyle
- un groupement hétérocyclique en C₅-C₁₀, préférentiellement en C₅-C₆, comportant de 1 à 6 hétéroatomes choisis parmi les éléments de la famille des chalcogènes et des pnictogènes, et
- un groupe cycloalkyle en C₃-C₆,
ledit groupe B étant substitué par un ou plusieurs substituant(s), préférentiellement par un substituant, le(s)dit(s) substituant(s) étant choisi(s) dans le groupe comprenant :
∘ un atome d'halogène,
∘ un groupe alcoxy comprenant de 1 à 4 atomes de carbone,
∘ un groupe aryloxy, notamment un groupe phényle oxy
∘ un groupe alkyle linéaire, ramifié ou cyclique, saturé ou insaturé, comprenant de 1 à 6 atomes de carbone, de préférence de 2 à 4 atomes de carbone, éventuellement substitué par un ou plusieurs atomes d'halogènes, et
∘ un groupe aryle, préférentiellement un groupe phényle e, éventuellement substitué par un alkyle en C₁-C₄,
∘ un groupe hydroxyle,
∘ un groupe thiol,
∘ un groupe amine,
∘ un groupe amide,
∘ un groupe acide carboxylique,
∘ un groupe cyano,
∘ un groupe azoture, et
∘ un groupe nitro,
lesdits composés étant sous la forme, d'un racémate, de l'un quelconque de leurs énantiomères, ou de l'un quelconque des différents tautomères correspondant aux dits racémates et énantiomères, ainsi que sous la forme de leurs sels pharmaceutiquement acceptables.

2. Composés selon la revendication 1, de formule générale (Ib) suivante : 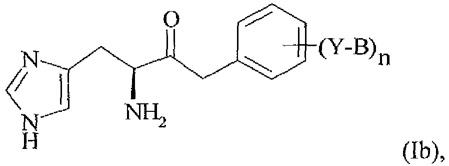 où n varie de 1 à 5, ledit groupement Y-B étant tel que défini dans la revendication 1.

3. Composés selon la revendication 2, **caractérisés en ce que**, lesdits composés sont monosubstitués par le groupe Y-B, ledit groupe Y-B étant tel que :
- Y représente un alkylène en C₁-C₃, un alcénylène en C₂-C₃ ou un alcynylène en C₂-C₃, et
- B représente un groupe aryle ou hétéroaryle, préférentiellement un groupe phényle, substitué par :
∘ un atome d'halogène,
∘ un groupe alcoxy comprenant de 1 à 4 atomes de carbone,
∘ un groupe aryloxy, notamment un groupe phényle oxy
∘ un groupe alkyle linéaire, ramifié ou cyclique, saturé ou insaturé, comprenant de 1 à 6 atomes de carbone, de préférence de 2 à 4 atomes de carbone, éventuellement substitué par un ou plusieurs atomes d'halogènes, et
∘ un groupe aryle, préférentiellement un groupe phényle e, éventuellement substitué par un alkyle en C₁-C₄.
∘ un groupe hydroxyle,
∘ un groupe thiol,
∘ un groupet amine, primaire secondaire ou ternaire
∘ un groupe amide,
∘ un groupe acide carboxylique,
∘ un groupe cyano,
∘ un groupe azoture, et
∘ un groupe nitro.

4. Composés selon la revendication 2 ou 3, dans lesquels Y-B est en position para du groupe phényle.

5. Composés selon l'une quelconque des revendications précédentes, lesdits composés étant choisis parmi les composés suivants :

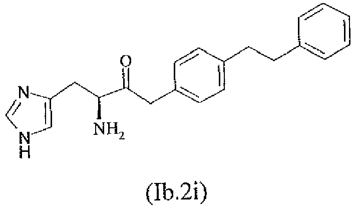

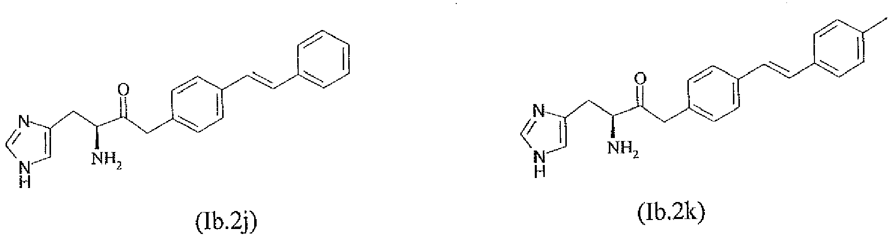

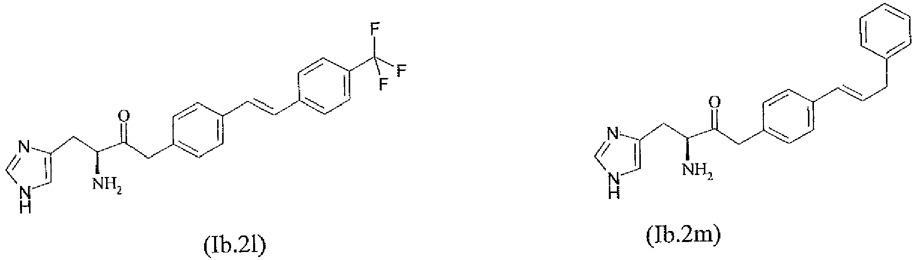

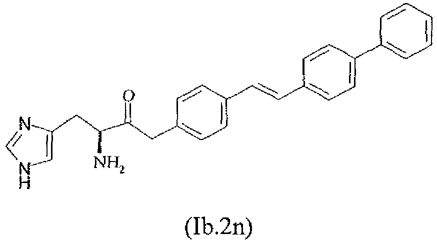

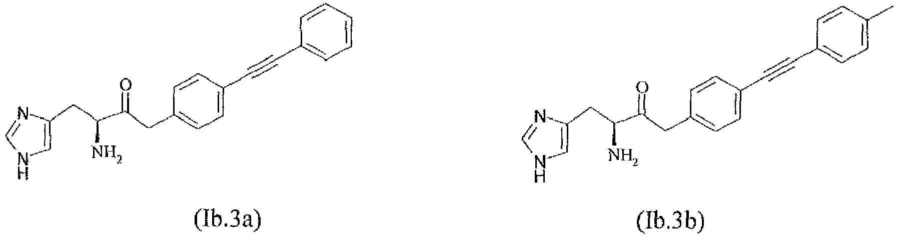

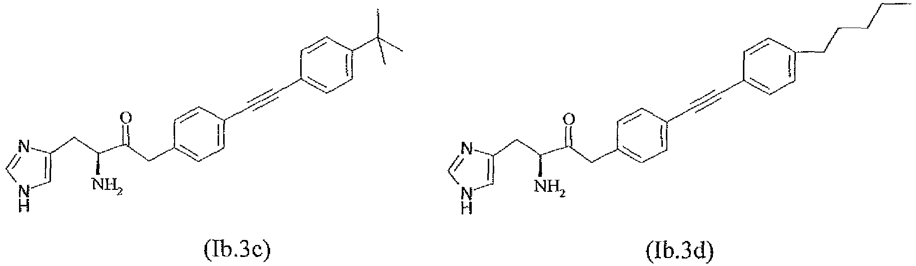

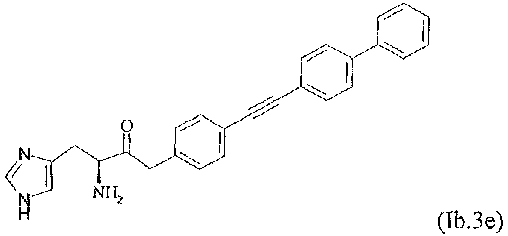

6. Composés selon l'une quelconque des revendications précédentes, à titre de médicament.

7. Compositions pharmaceutiques comprenant à titre de substance active au moins un composé selon l'une quelconque des revendications 1 à 5, en association avec un véhicule pharmaceutiquement acceptable.

8. Composés selon l'une quelconque des revendications 1 à 6, ou compositions pharmaceutiques selon la revendication 7, pour leur utilisation dans le traitement ou la prévention d'infections causées par des microorganismes.

9. Composés ou compositions pharmaceutiques pour leur utilisation selon la revendication 8, où lesdits microorganismes sont des microorganismes exprimant l'enzyme histidinol deshydrogénase.

10. Composés ou compositions pharmaceutiques pour leur utilisation la revendication 8 ou 9, où lesdits microorganismes sont des bactéries, notamment des bactéries des genres *Brucella* ou *Mycobacterium*

## Patentansprüche

1. Verbindungen der folgenden allgemeinen Formel (Ia): 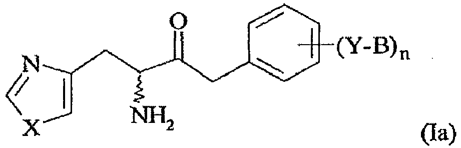 wobei X ein N darstellt,
**dadurch gekennzeichnet, dass**
- die Phenyl-Gruppe durch n Gruppe(n) Y-B substituiert ist, wobei n von 1 bis 5 variiert, wobei die Gruppe Y-B derart ist, dass:
- Y eine lineare oder verzweigte, gesättigte oder ungesättigte, Alkylen-Gruppe mit 1 bis 6 Kohlenstoffatomen, vorzugsweise 2 bis 4 Kohlenstoffatomen, darstellt, und
- B eine Gruppe darstellt, ausgewählt aus:
- einer Aryl- oder Heteroaryl-Gruppe, insbesondere einer Phenyl-Gruppe e oder Naphthyl-Gruppe,
- einer heterocyclischen C₅-C₁₀-, bevorzugt C₅-C₆-, Gruppe mit 1 bis 6 Heteroatomen, ausgewählt aus den Elementen der Familie der Chalcogene und der Pnictogene, und
- einer C₃-C₆-Cycloalkyl-Gruppe, wobei die Gruppe B durch einen oder mehrere Substituenten, bevorzugt durch einen Substituenten, substituiert ist, wobei der (die) Substituenten ausgewählt ist (sind) aus der Gruppe umfassend:
∘ ein Halogenatom,
∘ eine Alkoxy-Gruppe mit 1 bis 4 Kohlenstoffatomen,
∘ eine Aryloxy-Gruppe, insbesondere eine Phenyloxy-Gruppe,
∘ eine lineare, verzweigte oder cyclische, gesättigte oder ungesättigte, Alkyl-Gruppe mit 1 bis 6 Kohlenstoffatomen, vorzugsweise 2 bis 4 Kohlenstoffatomen, gegebenenfalls substituiert durch ein oder mehrere Halogenatome, und
∘ eine Aryl-Gruppe, bevorzugt eine Phenyl-Gruppe e, gegebenenfalls substituiert durch ein C₁-C₄-Alkyl,
∘ eine Hydroxyl-Gruppe,
∘ eine Thiol-Gruppe,
∘ eine Amin-Gruppe,
∘ eine Amid-Gruppe,
∘ eine Carbonsäure-Gruppe,
∘ eine Cyano-Gruppe,
∘ eine Azid-Gruppe, und
∘ eine Nitro-Gruppe,
wobei die Verbindungen in der Form eines Racemats, eines ihrer Entiomere oder eines der verschiedenen Tautomere, die den Racematen und Enantiomeren entsprechen, sowie in der Form eines ihrer pharmazeutisch annehmbaren Salze vorliegen.

2. Verbindungen nach Anspruch 1 der folgenden allgemeinen Formel (Ib): 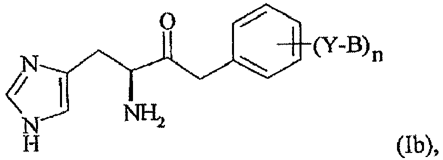 wobei n von 1 bis 5 variiert, wobei die Gruppe Y-B wie in Anspruch 1 definiert ist.

3. Verbindungen nach Anspruch 2, **dadurch gekennzeichnet, dass** die Verbindungen durch die Gruppe Y-B monosubstituiert sind, wobei die Gruppe Y-B derart ist, dass:
- Y ein C₁-C₃-Alkylen, ein C₂-C₃-Alkenylen oder ein C₂-C₃-Alkynylen darstellt, und
- B eine Aryl- oder Heteroaryl-Gruppe, bevorzugt eine Phenyl-Gruppe, darstellt, substituiert durch:
∘ ein Halogenatom,
∘ eine Alkoxy-Gruppe mit 1 bis 4 Kohlenstoffatomen,
∘ eine Aryloxy-Gruppe, insbesondere eine Phenyloxy-Gruppe,
∘ eine lineare, verzweigte oder cyclische, gesättigte oder ungesättigte, Alkyl-Gruppe mit 1 bis 6 Kohlenstoffatomen, vorzugsweise 2 bis 4 Kohlenstoffatomen, gegebenenfalls substituiert durch ein oder mehrere Halogenatome,
∘ eine Aryl-Gruppe, bevorzugt eine Phenyl-Gruppe e, gegebenenfalls substituiert durch ein C₁-C₄-Alkyl,
∘ eine Hydroxyl-Gruppe,
∘ eine Thiol-Gruppe,
∘ eine primäre, sekundäre oder ternäre Amin-Gruppe,
∘ eine Amid-Gruppe,
∘ eine Carbonsäure-Gruppe,
∘ eine Cyano-Gruppe,
∘ eine Azid-Gruppe, und
∘ eine Nitro-Gruppe.

4. Verbindungen nach Anspruch 2 oder 3, wobei Y-B in para-Position der Phenyl-Gruppe vorliegt.

5. Verbindungen nach einem der vorhergehenden Ansprüche, wobei die Verbindungen aus den folgenden Verbindungen ausgewählt sind:

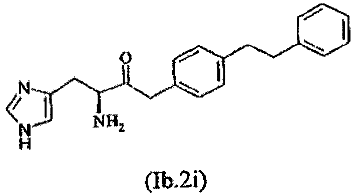

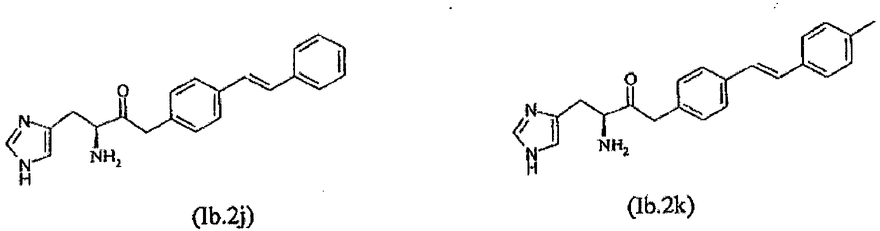

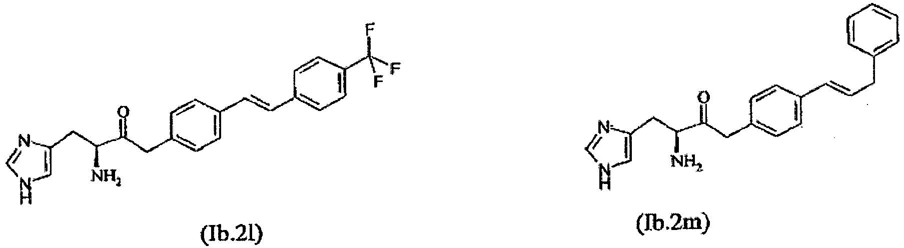

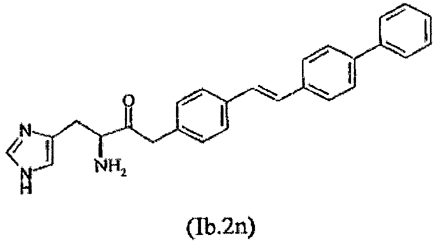

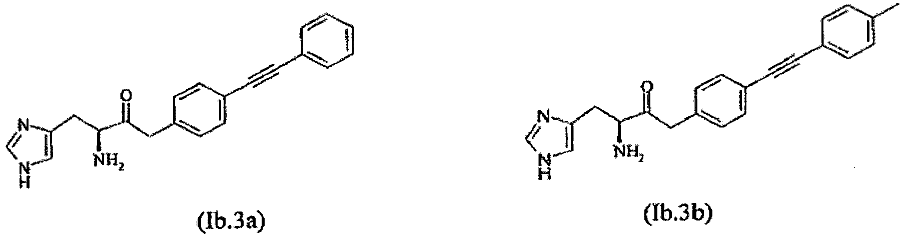

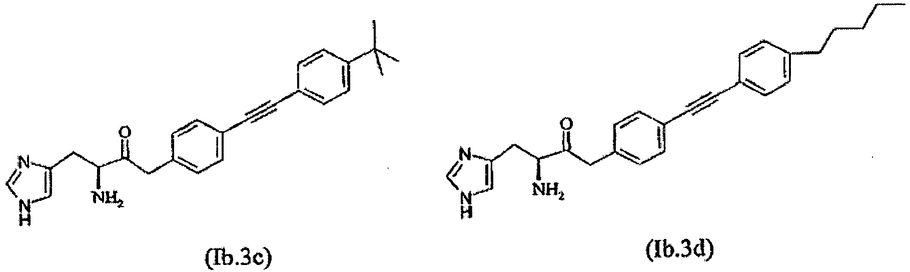

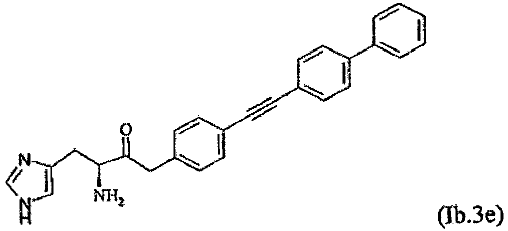

6. Verbindungen nach einem der vorhergehenden Ansprüche, als Medikamente.

7. Pharmazeutische Zusammensetzungen, umfassend als aktive Substanz mindestens eine Verbindung nach einem der Ansprüche 1 bis 5 in Assoziation mit einem pharmazeutisch annehmbaren Träger.

8. Verbindungen nach einem der Ansprüche 1 bis 6, oder pharmazeutische Zusammensetzungen nach Anspruch 7, zur Verwendung bei der Behandlung oder der Prävention von durch Mikroorganismen verursachten Infektionen.

9. Verbindungen oder pharmazeutische Zusammensetzungen zur Verwendung nach Anspruch 8, wobei die Mikroorganismen das Enzym Histidinoldehydrogenase exprimierende Mikroorganismen sind.

10. Verbindungen oder pharmazeutische Zusammensetzungen zur Verwendung nach Anspruch 8 oder 9, wobei die Mikroorganismen Bakterien, insbesondere Bakterien der Gattungen *Brucella* oder *Mycobacterium,* sind.

## Claims

1. Compounds of following general formula (Ia) : 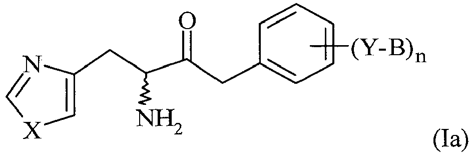 in which X is N
**characterized in that**
- the phenyl group is substituted by n Y-B group(s), n varying from 1 to 5, said Y-B group being such that:
- Y represents a linear or branched, saturated or unsaturated alkylene group, comprising from 1 to 6 carbon atoms, preferably from 2 to 4 carbon atoms, and
- B represents a group chosen from:
- an aryl or heteroaryl group, in particular a phenyl or naphthyl group
- a C₅-C₁₀ heterocyclic group, preferentially in C₅-C₆, comprising 1 to 6 heteroatoms chosen from the elements of the chalcogen and pnictogen families, and
- a C₃-C₆ cycloalkyl group,
said B group being substituted by one or more substituent(s), preferentially by one substituent, said substituent(s) being chosen from the group comprising:
∘ a halogen atom,
∘ an alkoxy group comprising from 1 to 4 carbon atoms,
∘ an aryloxy group, in particular a phenyloxy group
∘ a saturated or unsaturated, linear, branched or cyclic alkyl group, comprising from 1 to 6 carbon atoms, preferably from 2 to 4 carbon atoms, optionally substituted by one or more halogen atoms, and
∘ an aryl group, preferentially a phenyl group, optionally substituted by a C₁-C₄ alkyl,
∘ a hydroxyl group,
∘ a thiol group,
∘ an amine group,
∘ an amide group,
∘ a carboxylic acid group,
∘ a cyano group,
∘ an azide group, and
∘ a nitro group,
said compounds being in the form of a racemate, of any one of their enantiomers, or any one of the different tautomers corresponding to said racemates and enantiomers, as well as in the form of their pharmaceutically acceptable salts.

2. Compounds according to claim 1 of following general formula (Ib): 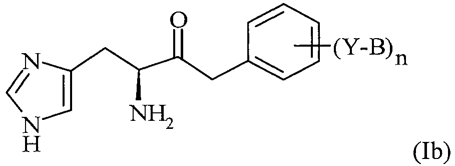 in which n varies from 1 to 5 and said Y-B group is as defined in claim 1.

3. Compounds according to claim 2, **characterized in that** said compounds are monosubstituted by the Y-B group, said Y-B group being such that:
- Y represents a C₁-C₃ alkylene, a C₂-C₃ alkenylene or a C₂-C₃ alkynylene, and
- B represents an aryl or heteroaryl group, preferentially a phenyl group, substituted by:
∘ a halogen atom,
∘ an alkoxy group comprising from 1 to 4 carbon atoms,
∘ an aryloxy group, in particular a phenyloxy group
∘ a saturated or unsaturated, linear, branched or cyclic alkyl group, comprising from 1 to 6 carbon atoms, preferably from 2 to 4 carbon atoms, optionally substituted by one or more halogen atoms, and
∘ an aryl group, preferentially a phenyl group, optionally substituted by a C₁-C₄ alkyl.
∘ a hydroxyl group,
∘ a thiol group,
∘ a primary, secondary or ternary amine group
∘ an amide group,
∘ a carboxylic acid group,
∘ a cyano group,
o an azide group, and
o a nitro group

4. Compounds according claim 2 or 3, in which Y-B is in the para position of the phenyl group.

5. Compounds according to any one of the previous claims, said compounds being chosen from the following compounds:

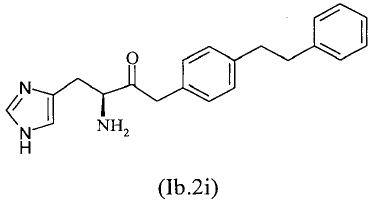

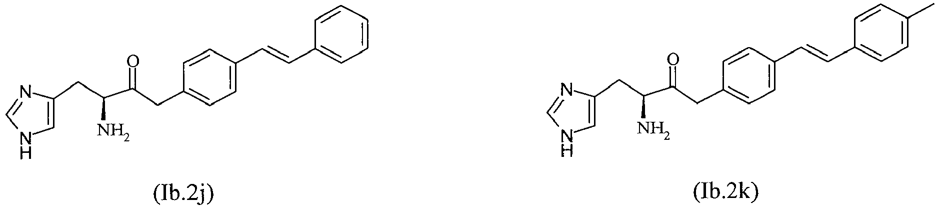

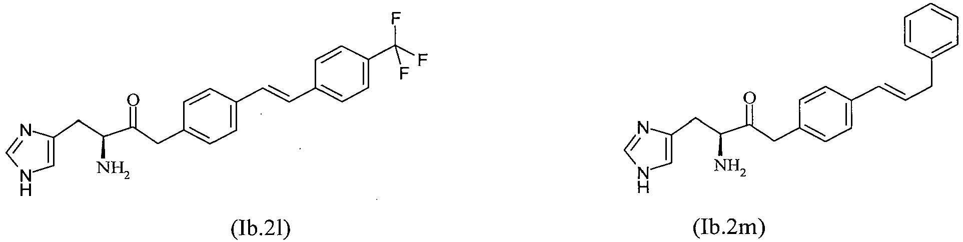

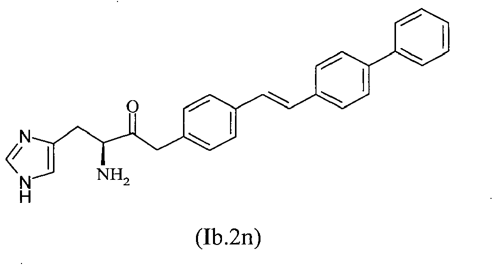

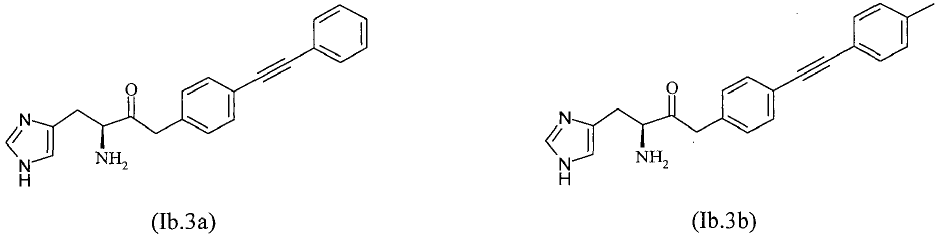

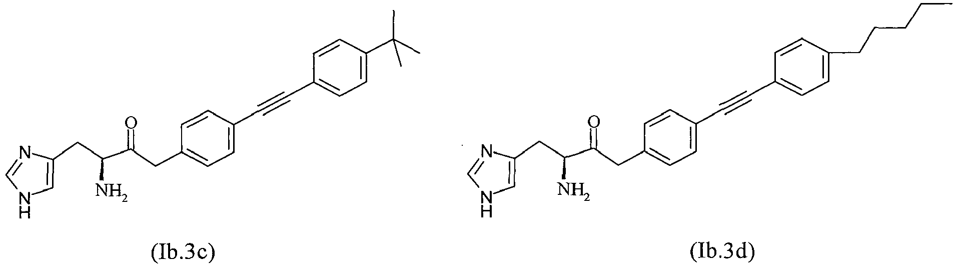

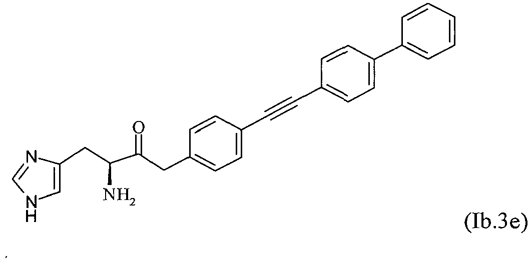

6. Compounds according to any one of the previous claims, as medicaments.

7. Pharmaceutical compositions comprising as active ingredient at least one compound according to any one of claims 1 to 5, in combination with a pharmaceutically acceptable vehicle.

8. Compounds according to any one of claims 1 to 6, or pharmaceutical compositions according to claim 7, for their use for the treatment or prevention of infections caused by microorganisms.

9. Compounds or pharmaceutical compositions for their use according to claim 8, where said microorganisms are microorganisms expressing the enzyme histidinol dehydrogenase.

10. Compounds or pharmaceutical compositions for their use according to claim 8 or 9, where said microorganisms are bacteria, in particular bacteria of the genera *Brucella* or *Mycobacterium.*
